# EUROPÄISCHE PATENTSCHRIFT

(11) **EP 1 282 827 B1**
(45) Veröffentlichungstag und Bekanntmachung des Hinweises auf die Patenterteilung: **26.01.2005**
(21) Anmeldenummer: 01933986.0
(22) Anmeldetag: 17.05.2001
(51) Int. Cl.: G01N 33/68, C12Q 1/48, C12N 9/12

(54) **VERFAHREN ZUM SELEKTIEREN VON INHIBITOREN FÜR ENZYME**
METHOD FOR SELECTING ENZYME INHIBITORS
PROCEDE DE SELECTION D'INHIBITEURS D'ENZYMES

(30) Priorität: 17.05.2000 DE 10024174
(43) Veröffentlichungstag der Anmeldung: 12.02.2003
(73) Patentinhaber: GSF-Forschungszentrum für Umwelt und Gesundheit GmbH, 85764 Oberschleissheim (DE)
(72) Erfinder: WARMUTH, Markus, 81241 München (DE); MATHES, Ruth, 81371 München (DE); HALLEK, Michael, 86938 Schondorf (DE)
(74) Vertreter: Grünecker, Kinkeldey, Stockmair & Schwanhäusser Anwaltssozietät
(86) Internationale Anmeldenummer: PCT/EP2001/005661
(87) Internationale Veröffentlichungsnummer: WO 2001/088530

(56) Entgegenhaltungen:
- WO-A-98/35048
- ERNEST ISABELLE ET AL: "Pyruvate kinase of Trypanosoma brucei: Overexpression, purification, and functional characterization of wild-type and mutated enzyme." PROTEIN EXPRESSION AND PURIFICATION, Bd. 13, Nr. 3, August 1998 (1998-08), Seiten 373-382, XP001066571 ISSN: 1046-5928
- IKEDA YOSHITAKA ET AL: "Allosteric regulation of pyruvate kinase M2 isozyme involves a cysteine residue in the intersubunit contact." JOURNAL OF BIOLOGICAL CHEMISTRY, Bd. 273, Nr. 20, 15. Mai 1998 (1998-05-15), Seiten 12227-12233, XP001066226 ISSN: 0021-9258
- UENO YOSHIHISA ET AL: "Regulatory phosphorylation of plant phosphoenolpyruvate carboxylase: Role of a conserved basic residue upstream of the phosphorylation site." FEBS LETTERS, Bd. 417, Nr. 1, 3. November 1997 (1997-11-03), Seiten 57-60, XP001066232 ISSN: 0014-5793
- BISHOP ANTHONY C ET AL: "Acquisition of inhibitor-sensitive protein kinases through protein design." PHARMACOLOGY & THERAPEUTICS, Bd. 82, Nr. 2-3, Mai 1999 (1999-05), Seiten 337-346, XP000974993 ISSN: 0163-7258
- BISHOP A C ET AL: "Generation of monospecific nanomolar tyrosine kinase inhibitors via a chemical genetic approach" JOURNAL OF THE AMERICAN CHEMICAL SOCIETY, AMERICAN CHEMICAL SOCIETY, WASHINGTON, DC, US, Bd. 121, Nr. 4, 1999, Seiten 627-631, XP002142032 ISSN: 0002-7863
- LIU Y ET AL: "STRUCTURAL BASIS FOR SELECTIVE INHIBITION OF SRC FAMILY KINASES BY PP1" CHEMISTRY AND BIOLOGY, CURRENT BIOLOGY, LONDON, GB, Bd. 6, Nr. 9, September 1999 (1999-09), Seiten 671-678, XP000978065 ISSN: 1074-5521
- BISHOP A C ET AL: "DESIGN OF ALLELE-SPECIFIC INHIBITORS TO PROBE PROTEIN KINASE SIGNALING" CURRENT BIOLOGY, CURRENT SCIENCE,, GB, Bd. 8, Nr. 5, 26. Februar 1998 (1998-02-26), Seiten 257-266, XP000974996 ISSN: 0960-9822
- WARMUTH MARKUS ET AL: "Genetic analysis of the ATP binding sites of Hck and ABL kinases reveal distinct and overlapping determinants for the specificity of the tyrosine kinase inhibitors PP1 and STI571." BLOOD, Bd. 96, Nr. 11 Part 1, 16. November 2000 (2000-11-16), Seite 510a XP001064435 42nd Annual Meeting of the American Society of Hematology;San Francisco, California, USA; December 01-05, 2000 ISSN: 0006-4971
- BOND CHRISTOPHER J ET AL: "Determinants of allosteric activation of yeast pyruvate kinase and identification of novel effectors using computational screening." BIOCHEMISTRY, Bd. 39, Nr. 50, 19. Dezember 2000 (2000-12-19), Seiten 15333-15343, XP001066224 ISSN: 0006-2960

## Beschreibung

Die Erfindung betrifft ein Verfahren zum Selektieren von Inhibitoren für Enzyme und ein Verfahren zum Prüfen eines Inhibitors auf dessen Spezifität in einem Modellsystem.

Herkömmlicherweise wird beim Screening nach neuen Leitstrukturen für Inhibitoren für Enzyme, beispielsweise Kinase-Inhibitoren, eine Substanzbibliothek gegen eine Vielzahl anderer Enzyme, wie Kinasen, getestet. Sodann werden die Leitstrukturen ausgewählt, die spezifisch das geprüfte Enzym, jedoch keines der anderen Enzyme inhibieren.

Ein solches Verfahren ist in Hanke et al., The Journal of Biological Chemistry, 1996, 271, S. 695 - 701 beschrieben. Dadurch wurden zwei Tyrosinkinase-Inhibitoren mit hoher Selektivität für Src-Kinasen im Vergleich zu einer Reihe anderer zellulärer Protein-Kinasen, wie den Kinasen ZAP70, Jak2, PKA und der EGF-Rezeptorkinase, gefunden. Diese Inhibitoren sind die nachstehenden Pyrazolopyrimidin-Derivate PP1 und PP2.

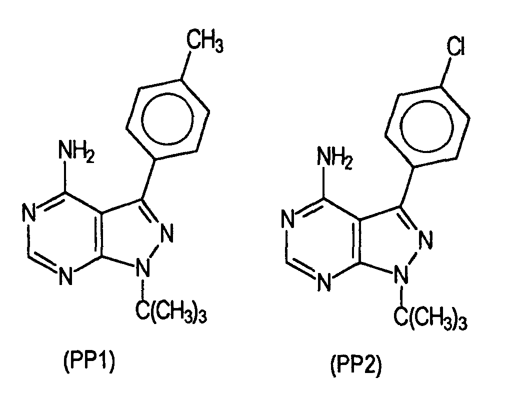

Außerdem wurde mit bekannten Screening-Verfahren gefunden, dass der Inhibitor STI571 (früher CGP57148) die Tyrosin-Kinase c-Abl spezifisch hemmt (Buchdunger et al, Cancer Research, 1996, 56, S. 100 -104).

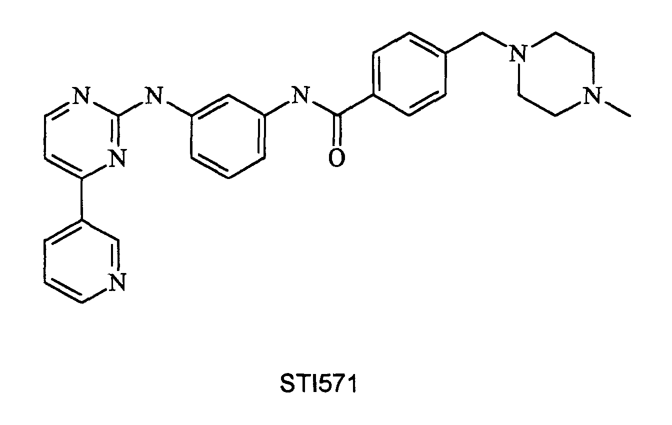

Diese Art des Screening ist schwierig durchzuführen, zeitaufwendig und teuer, insbesondere wegen der langwierigen Etablierung von dazu notwendigen Assay Arrays. Dabei muss jedes zu testende Enzym, beispielsweise eine Kinase, exprimiert und gereinigt werden. Anschliessend müssen für jedes Enzym, beispielsweise eine Kinase, die optimalen Reaktionsbedingungen herausgefunden werden. Die Entwicklungskosten und die Entwicklungszeit sind je nach Größe der Assay Arrays immens. Außerdem ist es nicht möglich, die biologische Aktivität der gefundenen Inhibitoren darauf zu prüfen, ob sie gegenüber der erwünschten Inhibierung eines bestimmten Enzyms spezifisch ist.

Chemistry & Biology 1999, Vol. 6, No. 6, S. 671 - 678 beschreibt Untersuchungen zur strukturellen Basis der Inhibitorspezifität von PP1 an Src Familiekinasen.

Aufgabe der vorliegenden Erfindung ist es daher, ein Verfahren zum Selektieren von Inhibitoren zur Verfügung zu stellen, das einfach, wenig zeitintensiv und kostengünstig ist.

Die Lösung dieser Aufgabe ist ein Verfahren zum Selektieren von Inhibitoren, die Schritte umfassend
a) Bestimmen einer Inhibitor- nicht aber Substrat-spezifischen Bindungsstelle in einer wt (Wildtyp) Proteinkinase,
b) Austausch mindestens einer Aminosäure an der als Inhibitor-spezifisch bestimmten Bindungsstelle der wt Proteinkinase durch eine von dieser verschiedenen Aminosäure, wodurch eine Mutante der wt Proteinkinase erhalten wird,
c) Prüfen der in Schritt b) erhaltenen Mutanten auf Enzym-Aktivität und Auswählen der aktiven Mutanten,
d) Prüfen mindestens einer Substanz mit der wt Proteinkinase und der in Schritt c) ausgewählten Mutante, und
e) Auswählen der Substanz als Inhibitor, wenn sie die wt Proteinkinase inhibiert, nicht aber die in Schritt c) ausgewählte Mutante.

Eine weitere der Erfindung zugrunde liegende Aufgabe ist es, ein einfaches und sicheres Verfahren zum Prüfen eines Inhibitors auf biologische Wirkungen, die für die Wechselwirkung zwischen dem Inhibitor und der Inhibitor-spezifischen Bindungsstelle spezifisch sind, zur Verfügung zu stellen.

Die Lösung dieser Aufgabe ist ein Verfahren zum Prüfen eines mit dem erfindungsgemäßen Verfahren selektierten Inhibitors auf biologische Wirkungen, die für die Wechselwirkung zwischen dem Inhibitor und der Inhibitor-spezifischen Bindungsstelle spezifisch sind, die Schritte umfassend
1) Inkubieren eines Modellsystems, in dem die wt Proteinkinase exprimiert wird, mit einem Inhibitor,
2) Feststellen der damit erzielten Wirkungen,
3) Inkubieren eines Modellsystems, in dem eine nach Schritt c) des erfindungsgemäßen Verfahrens selektierte Mutante exprimiert wird, mit dem Inhibitor,
4) Feststellen der damit erzielten Wirkungen,
5) Vergleichen der in Schritt 2) und 4) festgestellten Wirkungen, und
6) Auswählen der Wirkungen, die in Schritt 2) festgestellt wurden, nicht aber in Schritt 4), als spezifisch für die Wechselwirkung zwischen dem Inhibitor und der Inhibitor-spezifischen Bindungsstelle.

Die Erfindung wird nachstehend unter anderem anhand der Figuren erläutert.

Es zeigen,
Fig. 1 eine Darstellung des Bindungsmodus von PP1 im Vergleich zu Adenosin an die Bindungsstelle der Src-Kinase Hck;
Fig. 2 einen Vergleich der Aminosäuresequenz der Src-Kinase Hck im Bereich der PP1-Bindungsstelle mit einer Reihe anderer Protein-Kinasen;

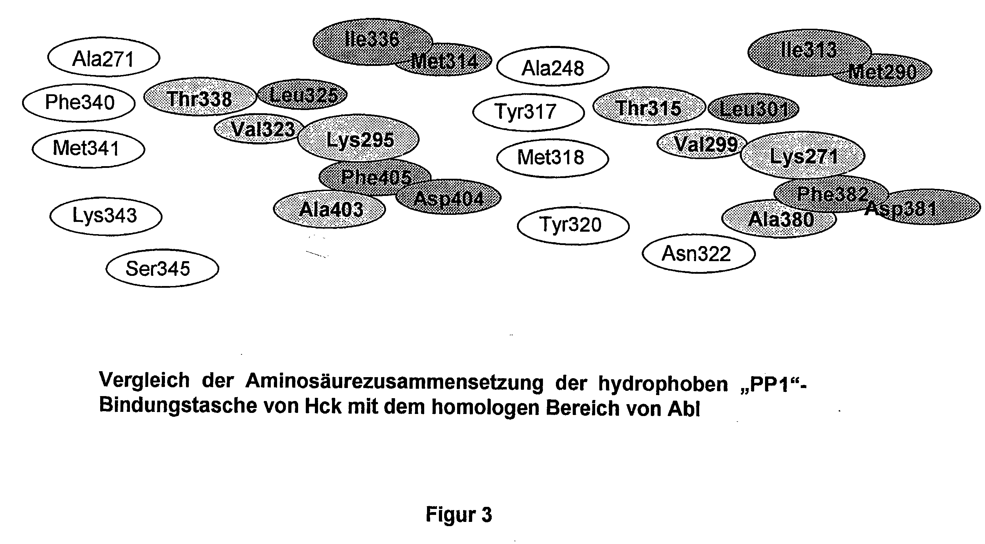

Fig. 3 einen Vergleich der Aminosäurezusammensetzung der hydrphoben "PP1"-Bindungstasche der Src-Kinase Hck mit dem homologen Bereich der Proteinkinase Abl
Fig. 4 einen Vergleich der Aminosäuresequenz der Proteinkinase Abl im Bereich einer potentiellen "Inhibitor"-Bindungstasche mit einer Reihe anderer Proteinkinasen;
Fig. 5 eine schematische Darstellung des Vorgehens bei einer Auswahl der herzustellenden Mutanten der Src-Kinase Hck an den Positionen 338 und 403;
Fig. 6 eine Zusammenfassung der an den Positionen 338 und 403 der Src-Kinase Hck durchgeführten Aminosäuresubstitutionen, wobei die eingeführten Mutationen zu einer sukzessiven Einengung des Zugangs zur hydrophoben PP1-Bindungstasche führen;
Fig. 7 eine schematische Darstellung des Vorgehens bei der Auswahl der herzustellenden Mutanten der Proteinkinase Abl an den Positionen 315 und 380;
Fig. 8 eine Zusammenfassung der an den Positionen 315 und 380 der Proteinkinase Abl durchgeführten Aminosäuresubstitutionen, wobei die eingeführten Mutationen zu einer sukzessiven Einengung des Zugangs zur hydrophoben "Inhibitor"-Bindungstasche führen;
Fig. 9 die Tyrosinphosphorylierungs-Aktivität der Mutanten T338V, T338L, T338I, T338M, T338Q, T338F, A403S, A403C und A403T im Vergleich zur wt Src-Kinase Hck und einer bekannten hyperaktiven Mutante, Hck Y501 F;
Fig. 10 die Tyrosinphosphorylierungs-Aktivität der Mutanten T315V, T315L, T315I, T315M, T315Q und T315F im Vergleich zur wt Bcr-Abl;
Fig. 11 eine schematische Darstellung der Selektion von spezifischen Inhibitoren mit Hilfe von mutierten Formen eines Enzyms;
Fig. 12 die Tyrosinphosphorylierungs-Aktivität der Mutanten T338V, T338L, T338I, T338M, T338Q und T338F im Vergleich zur wt Src-Kinase Hck mit (+) und ohne (-) Inkubation mit PP1;
Fig. 13 die Tyrosinphosphorylierungs-Aktivität der Mutanten A403S, A403C und A403T im Vergleich zur wt Src-Kinase Hck mit (+) und ohne (-) Inkubation mit PP1.
Fig. 14 die Tyrosinphosphorylierungs-Aktivität der Mutanten T315V, T315L, T315I, T315M, T315Q und T315F im Vergleich zur wt Bcr-Abl mit (+) und ohne (-) Inkubation mit PP1;

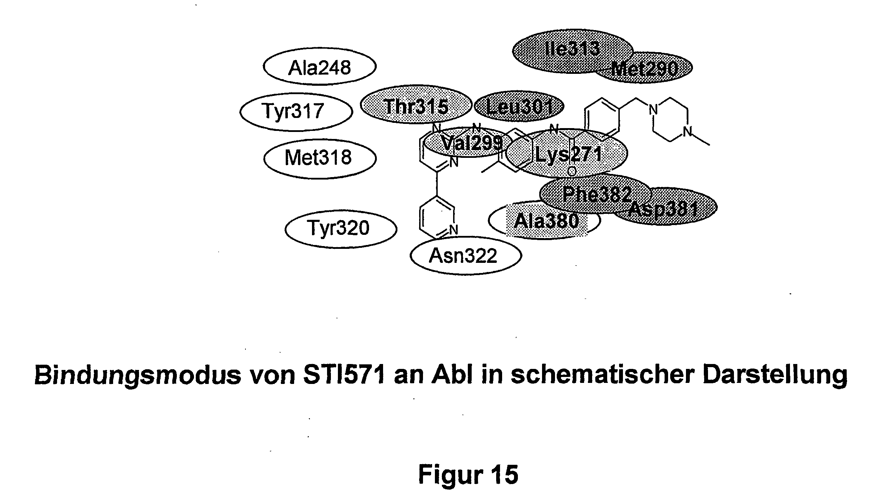

Fig. 15 eine schematische Darstellung des postulierten Bindungsmodus von STI571 an die Proteinkinase Abl;
Fig. 16 eine schematische Darstellung der biologische Prüfung eines Inhibitors mit Hilfe eines "knock-in"-Tiermodells, das eine inhibitor-resistente Mutante anstatt der wt Enzyms exprimiert;
Fig. 17 zwei Diagramme, die die Proliferation von STI571 oder PP1-behandelten 32D-Zellen, die entweder wt Bcr-Abl oder die Mutante Bcr-Abl T315M exprimieren, in Abwesenheit von Interleukin-3 als Überlebensfactor zeigen, wobei das Ausmaß der Proliferation als absolute Zellzahl dargestellt ist;
Fig. 18 zwei Diagramme, die das Überleben von STI571 - oder PP1-behandelten 32D-Zellen, die entweder wt Bcr-Abl oder die Mutante Bcr-Abl T315M exprimieren, in Abwesenheit von Interleukin-3 als Überlebensfaktor, zeigen, wobei das Ausmaß des Zelltods als Prozent Annexin-V-positiver Zellen dargestellt ist;
Fig. 19 eine schematische Darstellung der Ergebnisse aus den Figuren 17 und 18; und

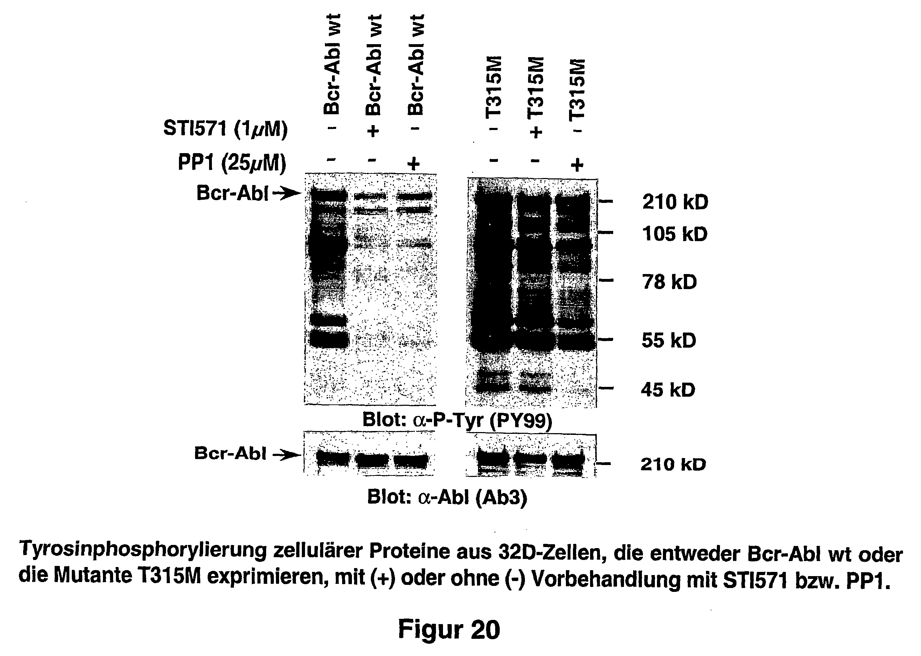

Fig. 20 die Tyrosinphosphorylierung zellulärer Proteine aus 32D-Zellen, die entweder wt Bcr-Abl oder die Mutante Bcr-Abl T315M exprimieren, mit (+) oder ohne (-) Vorbehandlung mit STI571 bzw- PP1.

Gemäß der vorliegenden Erfindung betrifft das Verfahren das Selektieren von Inhibitoren mit optimalen Bindungseigenschaften an ein bestimmtes, ausgewähltes Enzym, wobei spezifische Inhibitoren gefunden werden.

In Schritt a) des erfindungsgemäßen Verfahrens wird eine Inhibitor- nicht aber Substrat-spezifische Bindungsstelle in einer wt Proteinkinase bestimmt. Diese Bestimmung kann durch eine Analyse der Kristallstruktur einer wt Proteinkinase auf dessen Substrat-spezifische Bindungsstelle und der sterischen Anordnung eines bekannten Inhibitors für diese wt Proteinkinase durchgeführt werden. Dadurch kann die nur für den Inhibitor und nicht für das Substrat spezifische Bindungsstelle gefunden werden. Die Bestimmung einer Inhibitor- nicht aber Substrat-spezifischen Bindungsstelle kann auch ohne Kokristallisierung der wt Proteinkinase mit einem bekannten Inhibitor, also lediglich basierend auf der Kristallisierung des Enzyms mit seinem Substrat erfolgen. Außerdem kann statt der Kristallstruktur auch eine aufgrund der Aminosäuresequenz rechnerisch bestimmte Tertiärstruktur einer wt Proteinkinase verwendet werden.

Die in Schritt a) verwendete Proteinkinase ist vorzugsweise ausgewählt aus der Gruppe, bestehend aus den Src-Kinasen Src, Lyn, Fyn, Hck, Lck, Blk, Yes, Yrk, Fgr, den Kinasen ZAP70, BTK, Tec, Jak1, Jak2, PKA, VEGF-Familie-, PDGF-Familie- und EGF-Familie-Rezeptorkinasen, MAP-Kinasen, c-Abl und Zyklin-abhängigen Kinasen, insbesondere den Src-Kinasen Hck oder Lyn und der Kinase c-Abl.

Besonders bevorzugt werden in Schritt a) die Src-Kinase Hck und die Kinase Abl verwendet. Bei der nachstehenden Beschreibung werden der bekannten Inhibitor PP1 mit der Src-Kinase Hck und die bekannten Inhibitoren PP1 und STI571 mit der Tyrosin-Kinase Abl verwendet, wodurch gezeigt wird, dass mit dem erfindungsgemäßen Verfahren für die Enzyme spezifische Inhibitoren gefunden werden können In einer Ausführungsform wurden die von Schindler et al., Molecular Cell, 1999, 3, S. 638 - 648 und von Zhu et al., Structure, 1999, 7, S. 651 - 616 veröffentlichten Kristallstrukturen der Src-Kinase Hck im Komplex mit PP1 und der Src-Kinase Lck im Komplex mit PP2 genutzt, um die strukturellen Voraussetzungen für die Spezifität der Inhibitoren für die Src-Kinasen zu bestimmen.

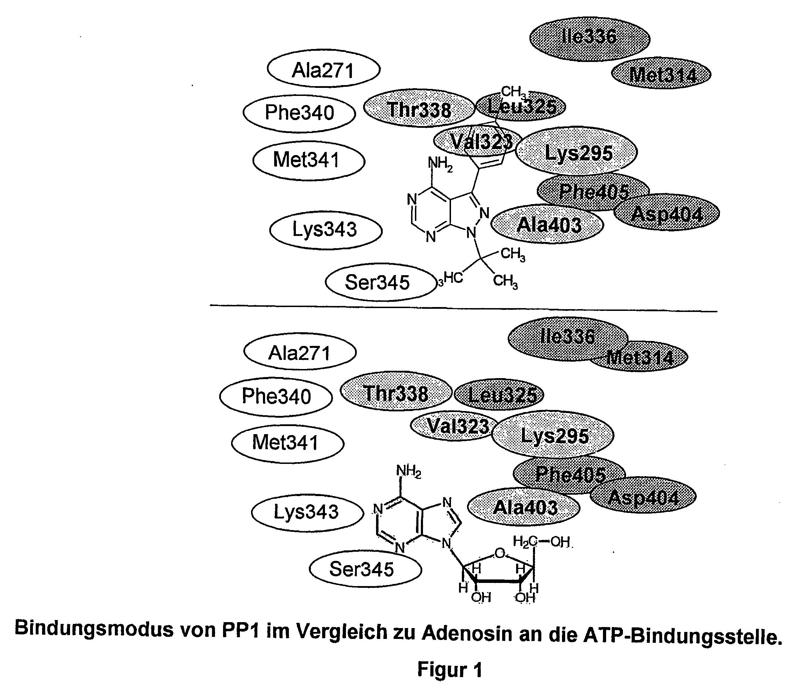

Beide Inhibitoren binden an die ATP-Bindungsstelle der Kinase-Domäne und verdrängen kompetitiv das für die Substratphosphorylierung wichtige ATP. Dabei entspricht der Bindungsmodus des Pyrazolopyrimidin-Gerüsts in etwa dem des Purin-Gerüsts des ATP. Zusätzlich aber schiebt sich der 4-Methylphenylrest von PP1 bzw. der 4-Chlorophenylrest von PP2 in eine hydrophobe Tasche, deren Eingang durch die Aminosäureseitenketten von Lysin (K) an Position 295, Valin (V) an Position 323, Threonin (T) an Position 338 und Alanin (A) an Position 403 begrenzt wird, wie in Figur 1 dargestellt. Daher ist diese hydrophobe Tasche eine Inhibitor- nicht aber Substrat-spezifische Bindungsstelle.

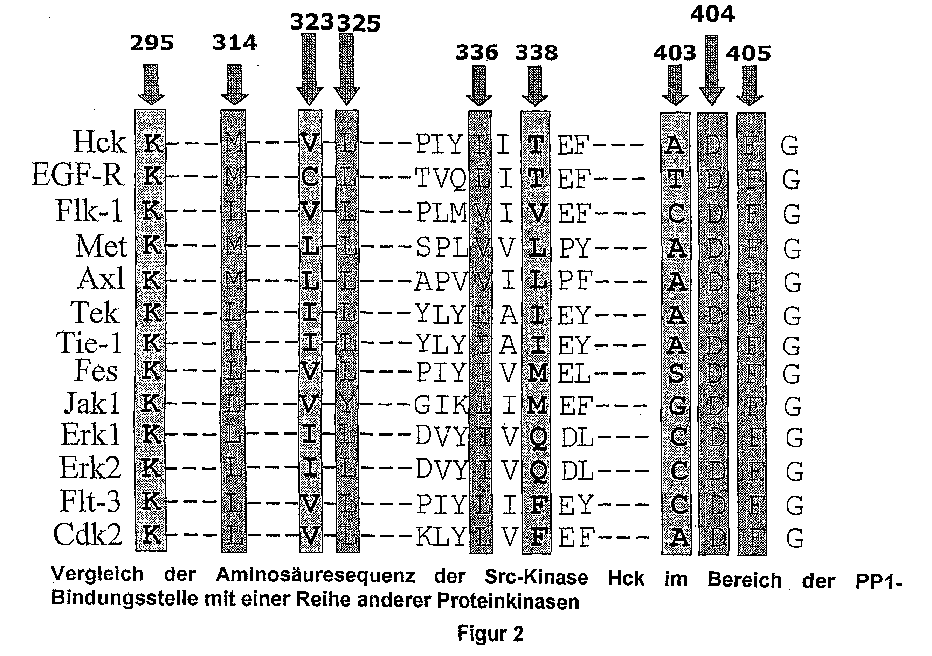

Sequenzvergleiche mit anderen Proteinkinasen -, z.B.Flk1, Met, Tie1, Jak1, Erk1 oder Flt3, zeigen, dass diese insbesondere an den jeweils zu T338 und A403 homologen Positionen andere Aminosäuren haben, wie in Figur 2 gezeigt. Beispielsweise findet man in Flt3-Kinase an der zu T338 homologen Position Phenylalanin statt Threonin und an der zu A403 homologen Position Cystein statt Alanin. Ein Vergleich der dreidimensionalen Strukturen von Hck und einer computersimulierten Mutante von Hck an Position 338 zu Phenylalanin zeigte, dass diese Unterschiede in der Aminosäuresequenz zu einer erheblichen Einengung des Zugangs zu der oben erwähnten hydrophoben Tasche führen. Diese Positionen dienen daher als sogenannte "molecular gatekeepers" bzw. Spezifitätsdeterminanten. Im Vergleich dazu zeigte sich zu den mit Lysin 295 und Valin 323 korrelierenden Positionen eine weitgehende Homologie innerhalb der Familie der Proteinkinasen.

Die hydrophobe Tasche selbst wird unter anderem begrenzt durch die Aminosäuren Methionin an Position 314, Leucin an Position 325, Isoleucin an Position 336, Aspartat an Position 404 und Phenylalanin an Position 405, wie ebenfalls in Figur 1 gezeigt. In ähnlicher Weise, wie für die Aminosäuren T338 und A403 beschrieben, beeinflussen Unterschiede in der Aminosäuresequenz die Raum- und Hydrophobizitätsverhältnisseinnerhalb der hydrophoben Tasche und somit die Bindung der Inhibitoren an die Bindungsstelle.

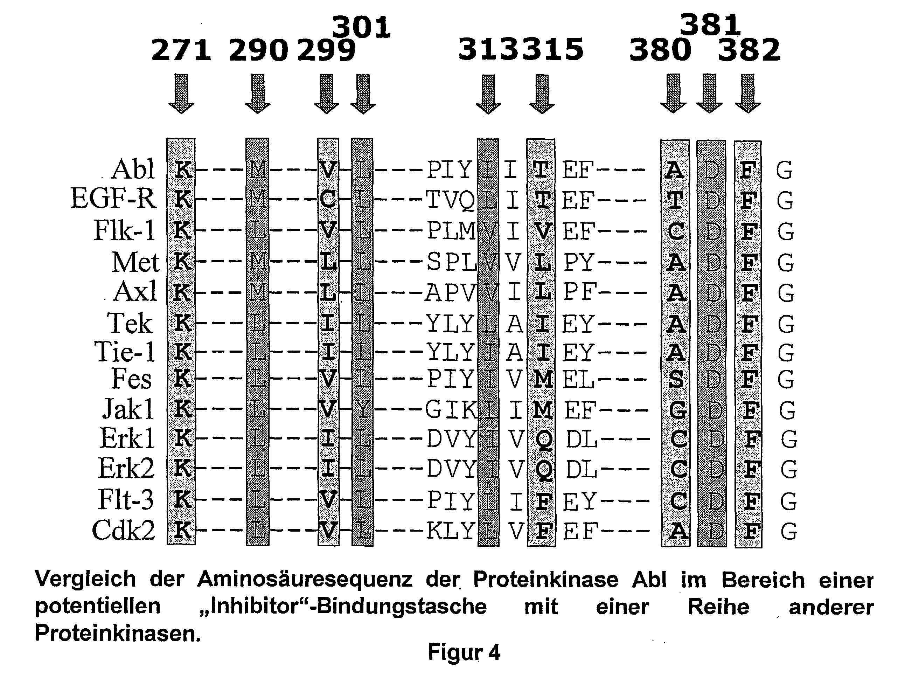

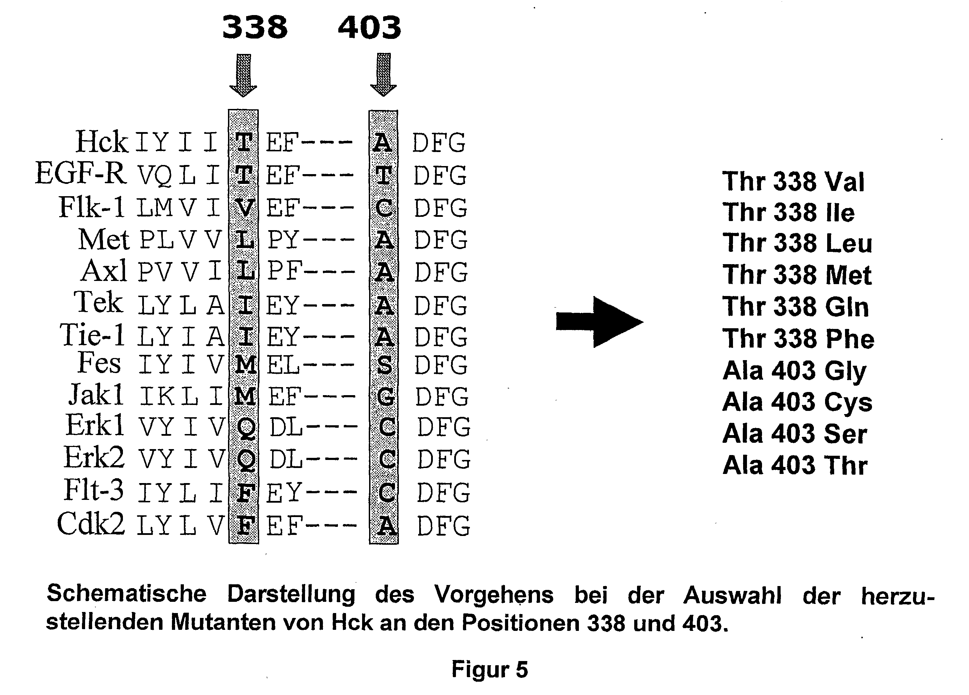

In einer anderen Ausführungsform wurde basierend auf Sequenzhomologievergleichen und einem berechneten Model eine potentielle Inhibitor-spezifische Bindungsstelle in der Tyrosinkinase Abl identifiziert. Dabei handelt es sich um eine Tasche, die zu der oben beschriebenen hydrophoben Tasche der Src Kinase Hck zu 100% homolog ist, wie den Fig.4 und 5 zu entnehmen. Der Eingangsbereich zu dieser Tasche wird durch die Aminosäuren Lysin271, Val299, Thr315 und Ala380 gebildet. Aminosäurealignments mit anderen Tyrosinkinasen zeigten, dass diese, wie bereits für Hck beschrieben, insbesondere an den jeweils zu T315 und A380 homologen Positionen andere Aminosäuren haben, wie in Figur 4 gezeigt. Diese Aminosäuren haben eine längere Seitenkette, so dass der Zugang zu oben beschriebender Tasche kritisch eingeschränkt oder verschlossen wird.

Somit wurde anhand zweier Ausführungsbeispiele gezeigt, wie mit Hilfe einer bekannten Kristallstruktur oder basierend auf Sequenzhomologievergleichen und einem berechneten Model die Inhibitor- nicht aber Substrat-spezifische Bindungsstelle in einem wt Enzym bestimmt werden kann.

In Schritt b) des erfindungsgemäßen Verfahrens wird mindestens eine Aminosäure an der als Inhibitor-spezifisch bestimmten Bindungsstelle durch eine davon verschiedene Aminosäure ausgetauscht. Die Anzahl der mutierten Positionen und die Art der im Austausch eingebrachten Aminosäuren hängt von der Struktur der als Inhibitor-spezifisch bestimmten Bindungsstelle ab. In der Regel werden vor allem solche Aminosäurepositionen verändert, die eine möglichst große Variabilität innerhalb einer Enzymfamilie aufweisen, d.h. innerhalb einer Enzymfamilie möglichst unterschiedlich mit Aminosäuren besetzt sind.

In Schritt b) wird die Aminosäure vorzugsweise durch eine sterisch aufwendigere, hydrophobere, hydrophilere, basischere oder azidere Aminosäure ausgetauscht.

Wenn die als Inhibitor-spezifisch bestimmte Bindungsstelle räumlich so verändert werden soll, dass der Zugang des Inhibitors an diese Bindungsstelle verhindert wird, ist es bevorzugt, Aminosäuren zum Austauschen zu wählen, die sterisch aufwendiger sind, als die ausgetauschte Aminosäure. Sterisch aufwendiger kann in diesem Zusammenhang bedeuten, dass die neue Aminosäure eine längere, bzw. voluminösere Seitekette hat, als die ausgetauschte. Es ist dabei bevorzugt pro Position mehrere Aminosäureaustausche durchzuführen, so dass eine graduelle Veränderung der räumlichen Verhältnisse im Bereich einer Inhibitor-spezifischen Bindungstelle entsteht. Solche Mutanten werden zu Bibliotheken zusammengefasst

Es ist außerdem möglich, die Eigenschaften der als Inhibitor-spezifisch bestimmten Bindungsstelle so zu verändern, dass die Hydrophobie oder Hydrophilie dieser Bindungsstelle verändert wird. Ist die Bindungsstelle beispielsweise mit überwiegend hydrophoben Aminosäuren aufgebaut, kann die Hydrophobie dadurch verändert werden, dass die neue Aminosäure hydrophil ist.

In einer Ausführungsform wurden in der Src-Kinase Hck an den Positionen 338 und 403 verschiedenste Aminosäure-Substitutionen durchgeführt, wie in Fig. 5 gezeigt. Die Nummerierung der Positionen ist auf die homologe Position in c-Src aus Huhn bezogen. Die neuen Aminosäuren sind dabei sterisch aufwendiger, als die in der wt Src-Kinase Hck vorhandenen. Diese Positionen sind, wie in der Analyse der Kristallstrukturen bestimmt und in Fig. 1 gezeigt, am Eingang der hydrophoben Tasche, die die Inhibitor-spezifische Bindungsstelle ist. Dadurch, dass die neuen Aminosäuren sterisch aufwendiger sind, wird der Zugang zu der hydrophoben Tasche verkleinert. Die Auswahl der durch Austausch eingebrachten Aminosäuren beruht dabei auf Sequenzhomologievergleichen, d.h. es wurden nur solche Aminosäuren eingebracht, die in anderen Kinasen an der homologen Position vorkommen. Bevorzugt werden dabei Kinasen aus der gleichen Spezies, also beispielsweise dem Menschen, berücksichtigt.

In einer Ausführungsform wurde in Schritt b) die Src-Kinase Hck verwendet, wobei vorzugsweise Threonin an der Position 338 und Alanin an der Position 403, durch eine davon verschiedene Aminosäure, ersetzt wurden.

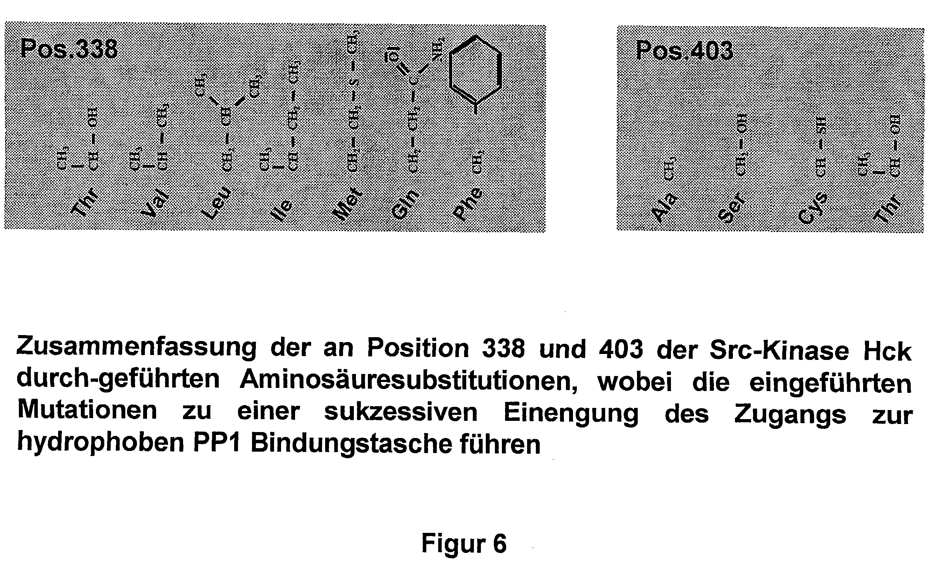

Vorzugsweise wird Threonin an der Position 338 ausgetauscht durch eine Aminosäure, ausgewählt aus der Gruppe, bestehend aus Valin, Leucin, Isoleucin, Methionin, Glutamin und Phenylalanin, und Alanin an der Position 403 durch eine Aminosäure, ausgewählt aus der Gruppe, bestehend aus Serin, Cystein und Threonin, Dies sind die Aminosäuren die an der homolgen Position in anderen Kinasen gefunden werden. Die entsprechenden Mutationen führen zu einer graduellen Einengung des Zugangs zur hydrophoben Tasche, wie in Fig. 6 gezeigt.

Außerdem kann in der Src-Kinase Hck Methionin an Position 314, Leucin an Position 325 und Isoleucin an Position 336 durch Phenylalanin und/oder Threonin ausgetauscht werden. Dadurch wird die Hydrophobie der hydrophoben Tasche einerseits durch die Substitution mit Threonin vermindert und der Raum innerhalb der Tasche durch die Substitution mit Phenylalanin verkleinert. Eine Mutation von Aspartat an Position 404 und Phenylalanin an Position 405 ist wegen deren wichtiger katalytischer Funktion nicht sinnvoll.

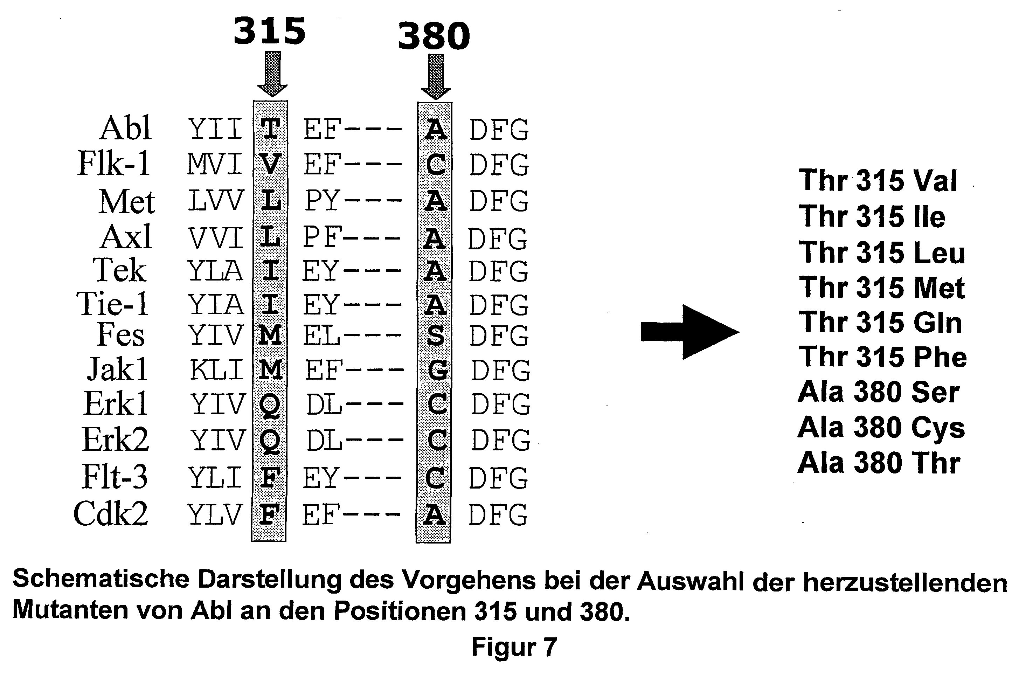

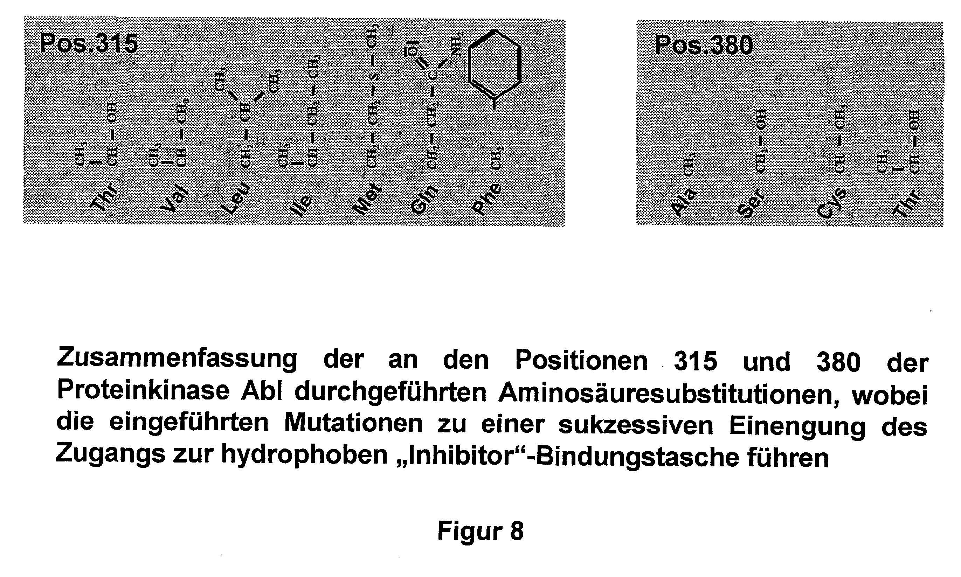

In einer anderen Ausführungsform wurde in der Tyrosinkinase Abl Threonin an der Position 315 gegen die Aminosäuren Valin, Leucin, Isoleucin, Methionin, Glutamin und Phenylalanin, ausgetauscht. Außerdem wurde in Abl Alanin an der Position 380 ausgetauscht gegen die Aminosäuren Serin, Cystein und Threonin . Dies sind die Aminosäuren die an der homolgen Position in anderen Kinasen gefunden werden, wie in Figur7 gezeigt. Die entsprechenden Mutationen führen zu einer graduellen Einengung des Zugangs des Zugangs zu der potentiellen Inhibitor-Bindungsstelle Tasche, wie in Fig. 8 gezeigt.

Außerdem kann in der Kinase Abl, Methionin an der Position 290, Leucin an der Position 301 und/oder Isoleucin an der Position 313 durch Phenylalanin und/oder Threonin ausgetauscht werden. Dadurch wird die Hydrophobie der hydrophoben Tasche einerseits durch die Substitution mit Threonin vermindert und der Raum innerhalb der Tasche durch die Substitution mit Phenylalanin verkleinert. Eine Mutation von Aspartat an Position 381 und Phenylalanin an Position 382 ist wegen deren wichtiger katalytischer Funktion nicht sinnvoll.

Der Austausch der Aminosäuren des Enzyms, beispielsweise der Src-Kinase Hck oder Lck, kann mit herkömmlichen Mutagenese-Verfahren, wie PCR-Mutagenese durchgeführt werden. Bei der PCR-Mutagenese wird das zu mutierende Gen in einen Klonierungsvektor eingebaut. Unter Verwendung von DNA-Primern, die mutagene Kodons enthalten, wird mit Hilfe einer Standard-PCR-Reaktion die entsprechende Mutation in das Gen eingebracht. Anschließend wird das mutierte Gen in Bakterien amplifiziert und die Mutation mittels Sequenzierung bestätigt.

Durch das Durchführen des Schritts b) werden Mutanten der wt Proteinkinase erhalten, die an den Inhibitor-spezischen Bindungsstellen sterisch oder in Bezug auf deren Hydrophilie, Hydrophobie, Basizität oder Azidität im Vergleich zur wt Proteinkinase verändert sind.

In Schritt c) des Verfahrens werden die in Schritt b) erhaltenen Mutanten daraufhin geprüft, ob sie noch die Enzym-Aktivität der wt Proteinkinase zeigen. Dann werden die Mutanten ausgewählt, bei denen der Austausch der Aminosäuren in Schritt b) die Enzym-Aktivität nicht beeinflusst, d.h. die aktiven Mutanten.

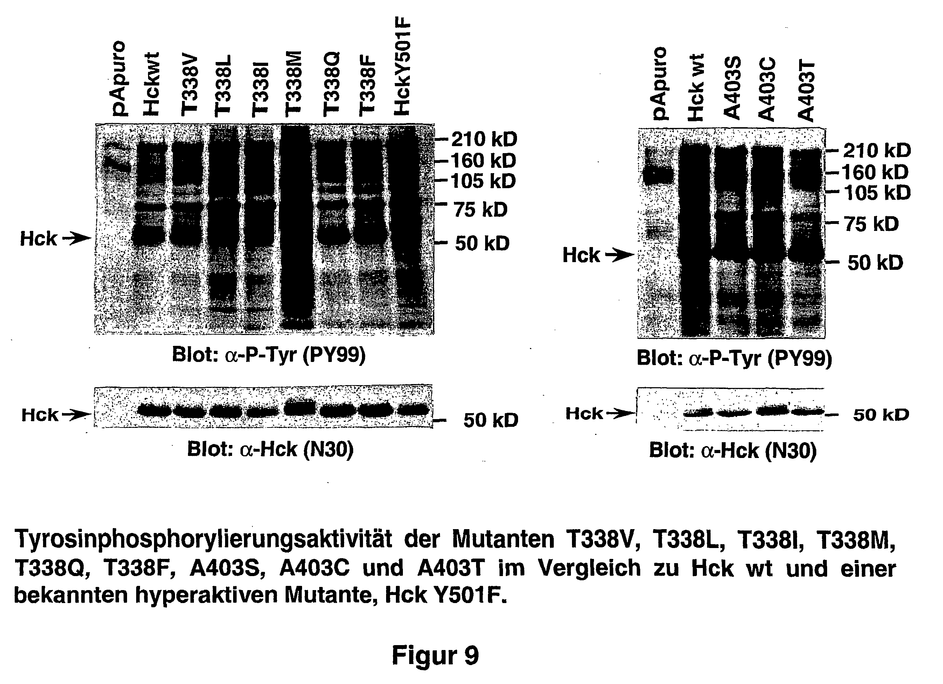

In einer Ausführungsform wurde die Tyrosinphosphorylierungs-Aktivität von Mutanten der Src-Kinase Hck geprüft, wie in Fig. 9 gezeigt. In den verwendeten Mutanten wurde jeweils Threonin an der Position 338 gegen Valin, Leucin, Isoleucin, Methionin, Glutamin und Phenylalanin ausgetauscht, und Alanin an der Position 403 gegen Serin, Leucin und Threonin. Die jeweiligen Mutanten wurden in Cos7-Zellen exprimiert.

Diese Zellen wurden lysiert und die so gewonnenen Proteinextrakte wurden anschließend im Western-Blot auf zelluläre Substrattyrosinphosphorylierung hin untersucht. Dies geschah unter Verwendung eines Antikörpers (PY99), der spezifisch tyrosinphosphorylierte Proteine erkennt. Das Ergebnis dieses Assays für die verschiedenen Mutationen an Position 338 und 403 ist in Figur 9 gezeigt. Wie in Figur 9 zu sehen, führt die Expression von wt Hck in Cos7-Zellen zur Induktion zahlreicher Phosphorylierungen. Drei Mutationen, T338L, T338I und T338M führen zu einer deutlichen Zunahme der durch Hck induzierten Phosphorylierung zellulärer Proteine. Diese Mutanten sind demnach aktiver als wt Hck. Fünf weitere Mutanten, T338V, T338Q ,T338F, A403S und A403C, zeigen in etwa gleiche Aktivität wie wt Hck. Eine Mutante, A403T, zeigte eine leicht geringere Aktivität als wt Hck.

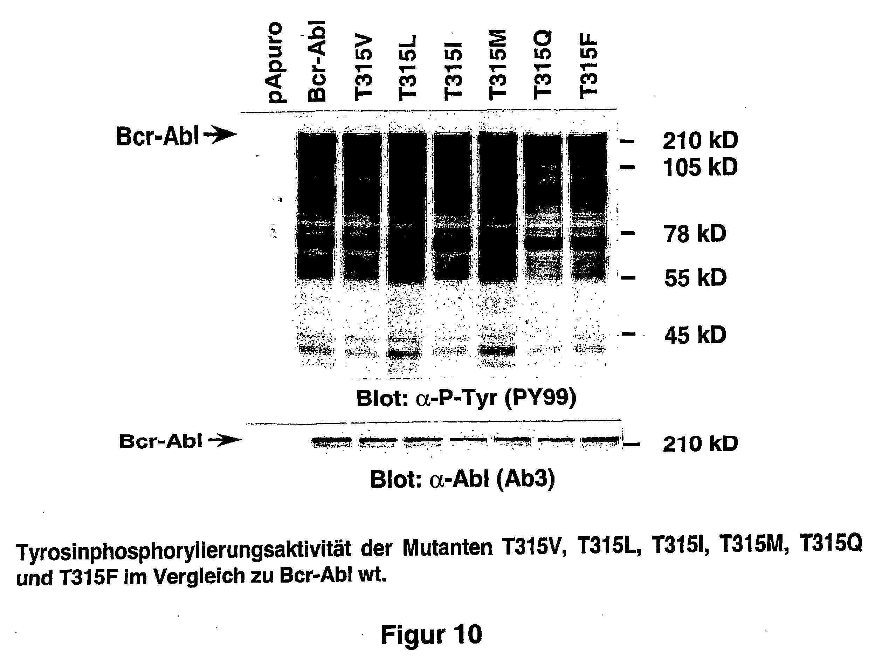

Somit ist der Fig. 9 zu entnehmen, dass ein Austausch von Threonin an Position 338 durch Leucin, Isoleucin und Methionin zu einer Hyperaktivierung und ein Austausch durch Valin, Glutamin und Phenylalanin zu einer der wt Src-Kinase Hck vergleichbaren Aktivität führt. Ebenfalls zu einer der wt Src-Kinase Hck vergleichbaren Aktivität führt der Austausch von Alanin an Position 403 zu Serin und Cystein. Ein Austausch zu Threonin führt zu einer geringfügigen Verringerung der Kinaseaktivität In einer weiteren Ausführungsform wurde die Tyrosinphosphorylierungs-Aktivität von Mutanten der Kinase Abl bzw. der leukämieinduzierenden Subfrom von Abl, Bcr-Abl, geprüft, wie in Fig. 10 gezeigt. In den verwendeten Mutanten wurde jeweils Threonin an der Position 315 gegen Valin, Leucin, Isoleucin, Methionin, Glutamin und Phenylalanin ausgetauscht. Die jeweiligen Mutanten wurden in Cos7-Zellen exprimiert.

Diese Zellen wurden lysiert und die so gewonnenen Proteinextrakte wurden anschließend im Western-Blot auf zelluläre Substrattyrosinphosphorylierung hin untersucht. Dies geschah wie oben unter Verwendung des Antikörpers PY99, der spezifisch tyrosinphosphorylierte Proteine erkennt. Das Ergebnis dieses Assays für die verschiedenen Mutationen an Position 315 ist in Fig.10 gezeigt. Wie in Fig. 10 zu sehen, führt die Expression von wt Bcr-Abl in Cos7-Zellen zur Induktion zahlreicher Phosphorylierungen. Alle in diesem Anwendungsbeispiel hergestellten Mutanten der Kinase Abl an Position 315 zeigen in etwa gleiche Tyrosinphosphorylierungsaktivität wie wt Bcr-Abl, bezogen auf die Phosphorylierung zellulärer Substrate durch die exprimierten Mutanten.

Fig.10 ist somit zu entnehmen, dass keiner der vorgenommenen Aminosäureaustausche an Position 315 die Tyrosinphosphorylierungseigenschaften von Bcr-Abl wesentlich verändert.

In Schritt c) können für die Durchführung der Schritte d) und e) des erfindungsgemässen Verfahrens alle Mutanten ausgewählt werden, welche enzymatische Aktivität besitzen. Es ist bevorzugt möglichst mehrere Mutanten mit einer möglichst graduellen Abstufung der Veränderungen an der für den Inhibitor spezifischen Bindungsstelle auszuwählen. Zeigt eine Mutante keinerlei Enzym-Aktivität mehr, kann davon ausgegangen werden, dass durch die Veränderung einer Aminosäure an der Inhibitor-spezifischen Bindungsstelle die tertiär Struktur des Enzyms so stark beeinflusst wurde, dass diese Mutante nicht mehr geeignet ist, in den nachfolgenden Schritten verwendet zu werden, um einen spezifischen Inhibitor für die wt Proteinkinase zu selektieren. Die in Schritt c) ausgewählte Mutante hat somit eine größtmögliche strukturelle Ähnlichkeit mit der wt Proteinkinase, mit dem Unterschied, dass an der für den Inhibitor spezifischen Bindungsstelle des Enzyms eine möglichst graduelle Veränderung vorgenommen wurde.

In einem Ausführungsbeispiel wurden für die Src-Kinase Hck die Mutanten T338V, T338L, T338I, T338M, T338Q, T338F, A403S, A403C und A403T selektiert. Diese Mutanten haben alle ausreichende Kinaseaktivität, um für die Schritte d) und c) verwendet zu werden.

In einem weiteren Ausführungsbeispiel wurden für die Kinase Abl die Mutanten T315V, T315L, T315I, T315M, T315Q und T315F selektiert. Diese Mutanten haben alle ausreichende Kinaseaktivität, um für die Schritte d) und c) verwendet zu werden.

Falls bereits ein spezifischer Inhibitor für ein Enzym bekannt ist, wie dies in den vorstehenden Ausführungsbeispielen der Fall ist, können in einer bevorzugten Ausführungsform die in Schritt c) der erfindungsgemäßen Verfahrens ausgewählten Mutanten zusätzlich darauf geprüft werden, ob diese durch den bekannten Inhibitor gehemmt werden. Ausgewählt werden sodann die Mutanten, die durch den bekannten Inhibitor nicht mehr gehemmt werden.

In Schritt d) des erfindungsgemäßen Verfahrens wird mindestens eine Substanz mit der wt Proteinkinase und den in Schritt c) ausgewählten Mutanten geprüft.

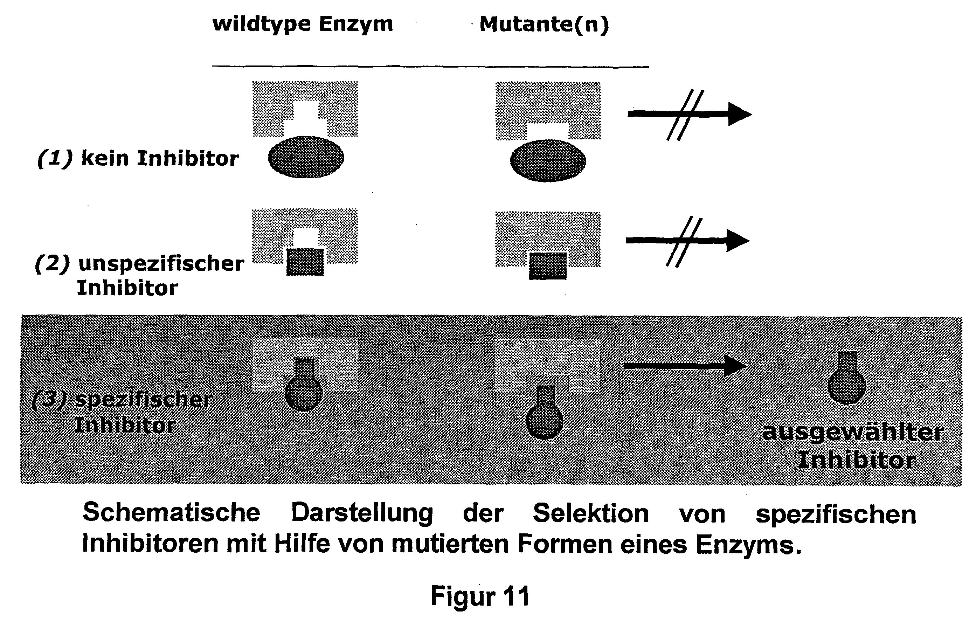

Vorzugsweise wird eine Substanzbibliothek aus verschiedensten Substanzen gleichzeitig geprüft. Dadurch werden 3 verschiedene Substanzklassen unterschieden, nämlich (1) Substanzen, die weder die wt Proteinkinase, noch die in Schritt c) ausgewählte Mutante inhibieren (keine Inhibitor), (2) Substanzen, die sowohl die wt Proteinkinase als auch die in Schritt c) ausgewählte Mutante inhibieren (unspezifischer Inhibitor) und (3) Substanzen, die nur die wt Proteinkinase, nicht aber die in Schritt c) ausgewählte Mutante inhibieren, wie in Fig. 11 schematisch dargestellt. Als Inhibitor werden sodann diejenigen Substanzen (3) ausgewählt, die nur die wt Proteinkinase, nicht aber die in Schritt c) ausgewählte Mutante inhibieren.

Vorzugsweise wird eine Substanz oder Substanzbibliothek mit einer Vielzahl von in Schritt c) ausgewählten Mutanten geprüft, vorzugsweise mit mindestens 2, insbesondere mit mindestens 5 und besonders bevorzugt mit mindestens 10 Mutanten. In dieser Ausführungsform wird bevorzugt diejenige Substanz ausgewählt, die nur die wt Proteinkinase und keine der ausgewählten Mutanten inhibiert.

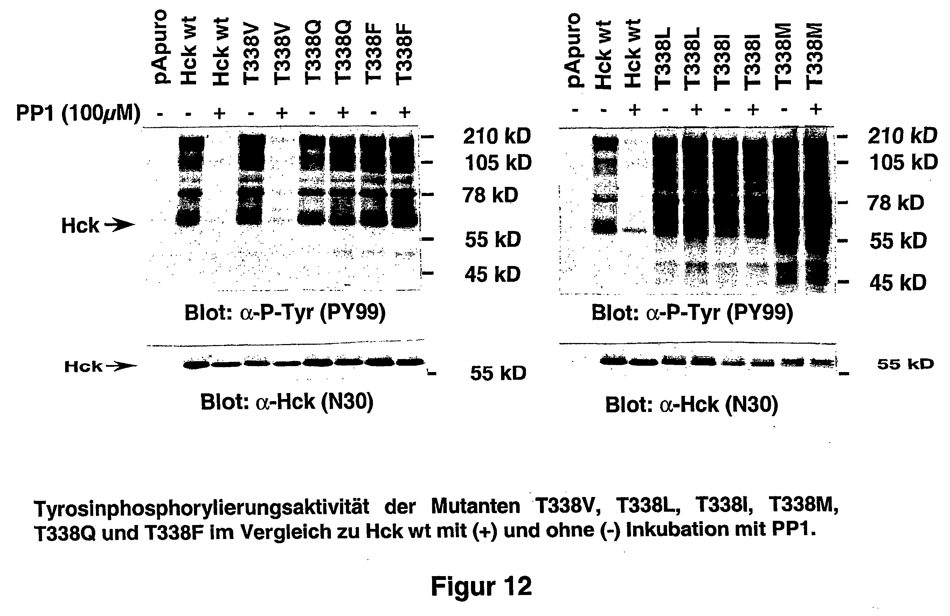

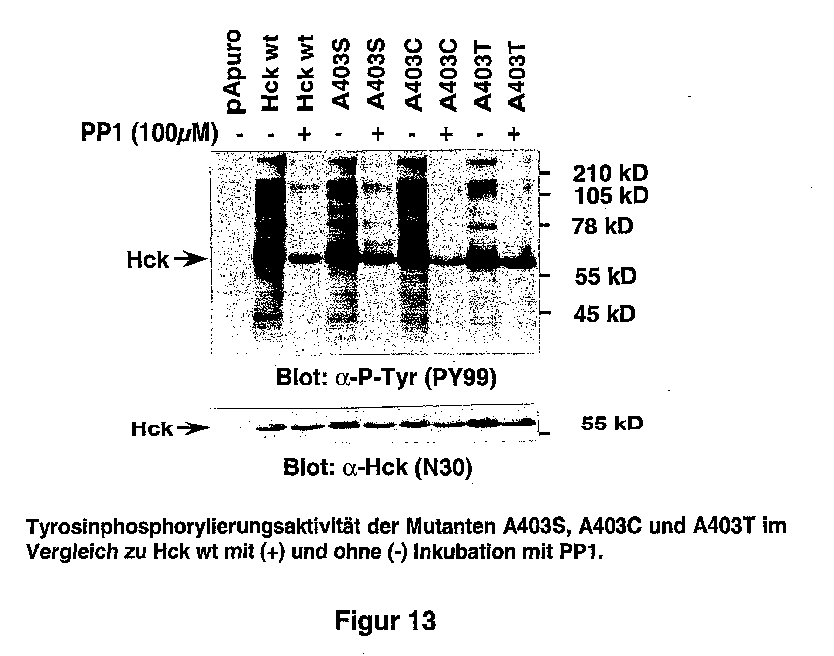

In einer Ausführungsform wurde überprüft ob mit dem erfindungsgemäßen Verfahren der bekannte Src-Inhibitor PP1 selektiert werden kann. Dazu wurde die Tyrosinphosphorylierungsaktivität der Mutanten, die durch den Austausch von Threonin and Position 338 durch Valin, Leucin, Isoleucin, Methionin, Glutamin und Phenylalanin und durch den Austausch von Alanin an Position 403 durch Serin, Cystein und Threonin in der wt Src-Kinase Hck erhalten wurden, mit und ohne die Zugabe des Inhibitors PP1 geprüft, wie in Fig. 12 und 13 gezeigt. Es ist den Fig. 12 und 13 zu entnehmen, dass die Mutanten Threonin zu Leucin, Isoleucin, Methionin, Glutamin und Phenylalanin nicht durch PP1 inhibiert wurden. Die Mutanten Threonin 315 zu Valin und Alanin 403 zu Serin, Cystein und Threonin wurden durch PP1 gehemmt wie die wt-Form von Hck. Dadurch wird PP1 als Substanz, welche in die hydrophobe Tasche, also die Inhibitor-spezifische Bindungsstelle, bindet selektiert. Dazu wurden, wie vorstehend für Schritt c) beschrieben, die Mutanten in Cos7-Zellen exprimiert, mit dem Unterschied, dass diese zusätzlich vor der Lyse mit PP1 inkubiert wurden.

Die Expression von wt Hck führt zu einer deutlichen Zunahme der Tyrosinphosphorylierung zahlreicher zellulärer Proteine. Die Inkubation der Zellen vor der Lyse mit 100µM PP1 hebt diese durch wt Hck induzierten Phosphorylierungen wieder auf bzw. reduziert sie deutlich. Gleiches gilt für die Mutanten T338V, A403S, A403C und A 403T. Im Gegensatz dazu bleiben die durch die Mutanten T338L, T338I, T338M, T338Q und T338F induzierten Phosphorylierungen auch nach Inkubation mit PP1 weitgehend unverändert. Diese Mutanten sind also resistent gegen eine Hemmung durch PP1. Daher wird durch eine ausreichende Einengung des Zugangs zur hydrophoben PP1-Bindungstasche durch Verlängerung der Aminosäureseitenkette an Position 338 eine Resistenz gegenüber PP1 induziert, ohne die Grundaktivität des Enzyms zu verlieren. Dieses Ergebnis bestätigt die Bedeutung der hydrophoben PP1-Bindungstasche für die Bindung von PP1. Umgekehrt führt eine Einengung des Zugangs zur hydrophoben "Inhibitor-Bindingstasche" durch Verlängerung der Aminosäureseitenkette an Position 403 nicht zu einer Resistenz gegenüber PP1.

In der nachstehenden Tabelle 1 sind Beispiele für durchgeführte Aminosäureustausche in der Kinase Hck und die Ergebnisse der in Schritt c) und d) durchgeführten Prüfungen zusammengefasst.

**Tabelle 1**

| Mutante | Kinase-Aktivität | durch PP1 inhibiert |
|---|---|---|
| wt | +++ | ja |
| Thr338Val | ++++ | ja |
| Thr338Leu | +++++ | nein |
| Thr338Ile | ++++ | nein |
| Thr338Met | ++++ | nein |
| Thr338Gln | +++ | nein |
| Thr338Phe | +++ | nein |
| Ala403Ser | +++ | ja |
| Ala403Cys | +++ | ja |
| Ala403Thr | -++ | ja |

Wie der Tabelle zu entnehmen ist, zeigten fünf der Mutanten, nämlich T338L T338I, T338M, T338Q und T338F, zwar noch Kinase-Aktivität, konnten durch PP1 jedoch nicht mehr inhibiert werden. Damit würde PP1 beim Durchführen des erfindungsgemäßen Verfahrens als Inhibitor ausgewählt werden.

In einer weiteren Ausführungsform wurde die Tyrosinphosphorylierungsaktivität der Tyrosin-Kinase Abl bzw. ihrer leukämieinduzierenden Form, Bcr-Abl, und den Mutanten von Bcr-Abl an Position 315 zu Valin, Leucin, Isoleucin, Methionin, Glutamin und Phenylalanin mit und ohne Zugabe bekannter Kinase-Inhibitoren getestet. Als Inhibitoren wurden dabei PP1 und STI571 verwendet. Dadurch sollten Inhibitoren bestimmt werden, die in die potentielle "Inhibitor-Bindungstasche" der Abl-Kinase binden und somit möglichst große Spezifität gegenüber anderen Proteinkinasen besitzen.

Dazu wurden, wie vorstehend für die Tyrosinkinase Hck beschrieben, die Mutanten in Cos7-Zellen exprimiert, mit dem Unterschied, dass diese zusätzlich vor der Lyse mit PP1 oder STI571 inkubiert wurden.

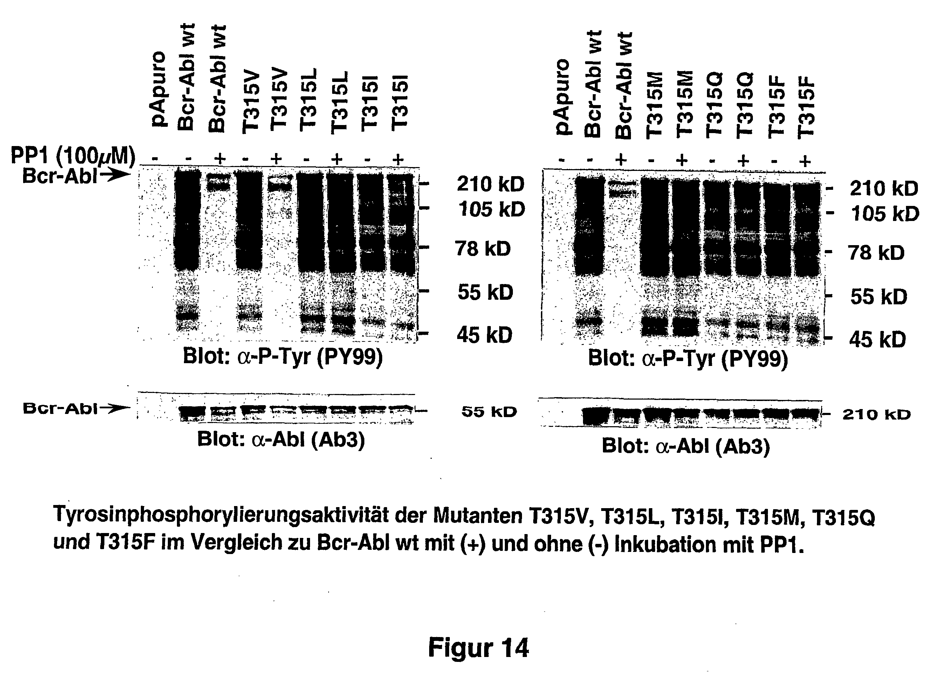

Figur 14 zeigt repräsentativ die Ergebnisse für die Inkubation von Bcr-Abl wt und den Mutanten von Bcr-Abl an der Position 315 zu Valin, Leucin, Isoleucin, Methionin, Glutamin und Phenylalanin mit PP1. Die Expression von Bcr-Abl wt führt zu einer deutlichen Zunahme der Tyrosinphosphorylierung zahlreicher zellulärer Proteine. Die Inkubation der Zellen vor der Lyse mit dem Inhibitor PP1 hebt diese durch Bcr-Abl wt induzierten Phosphorylierungen wieder auf bzw. reduziert sie deutlich. Ebenso aufgehoben werden die durch die Mutante T315V induzierten Tyrosinphosphorylierungen. Nicht aufgehoben durch PP1 werden hingegen die Tyrosinphosphorylierungen induziert durch die Mutanten T315L, T315I, T315M, T315Q und T315F. Diese Mutanten sind also resistent gegenüber PP1. Dies bestätigt, dass PP1 als Inhibitor in die hydrophobe "Inhibitor-Bindungstasche" von Bcr-Abl binden kann.

Der nachstehenden Tabelle 2 sind die Ergebnisse der Hemmung von Bcr-Abl und Mutanten von Bcr-Abl an den Positionen 315 und 380 mit PP1 und STI571 zu entnehmen.

**Tabelle 2**

| Mutante der Tyrosin-Kinase Abl | Kinase-Aktivität | Inhibierung durch PP1 | Inhibierung durch STI571 |
|---|---|---|---|
| Bcr-Abl wt | +++ | + | + |
| Thr 315 Val | +++ | + | - |
| Thr 315 Ile | +++ | - | - |
| Thr 315 Leu | +++ | - | - |
| Thr 315 Met | +++ | - | - |
| Thr 315 Gln | +++ | - | - |
| Thr 315 Phe | +++ | - | - |
| Ala 380 Ser | +++ | + | + |
| Ala 380 Cys | +++ | + | + |
| Ala 380 Thr | +++ | + | - |

Wie den Ergebnissen der Tabelle 2 zu entnehmen, hemmt PP1 noch die Mutanten T315V, A380S, A380C und A380T. Die Mutanten T315L, T315I, T315M, T315Q und T315F konnten nicht mehr durch PP1 inhibiert werden. Im Vergleich dazu hemmt STI571 nur noch die Mutanten A380S und A380C. Es konnten nicht mehr inhibiert werden die Mutanten T315V, T315L, T315I, T315M, T315Q und T315F sowie A380T. Damit wird bei ausreichender Verlängerung der Aminosäureseitenketten an Position 315 und 380 der Zugang für STI571 zur hydrophoben Inhibitor-Bindungstasche versperrt. Dies zeigt, dass STI571 ebenso wie PP1 in die hydrophobe "Inhibitor-Bindungstasche" von Bcr-Abl bindet und dass STI571 diese Tasche, basierend auf der graduellen Abstufung der Mutanten, besser ausfüllt als PP1. Daher wird mit dem erfindungsgemässen Verfahren der als sehr spezifisch für die Tyrosinkinase Abl bekannte und sich in klinischer Prüfung befindende Inhibitor STI571 im Vergleich zu PP1 selektiert.

In Schritt e) des Verfahrens wird die Substanz als Inhibitor ausgewählt, die die wt Proteinkinase inhibiert, nicht aber die in Schritt c) ausgewählten Mutanten, wie in Fig. 11 gezeigt. Bevorzugt ausgewählt werden dabei diejenigen Substanzen, die die wt Proteinkinase inhibieren, nicht aber diejenige Mutante mit der kleinsten strukturellen Veränderung, also beispielsweise mit einer nur geringfügigen Verlängerung der Aminosäureseitenkette an einer als Spezifitätsdeterminante identifizierten Position. Nach dem erfindungsgemäßen Verfahren wird in vorstehend beschriebenen Beispiel in Schritt e) jeweils PP1 als Inhibitor für die Src-Kinase Hck ausgewählt und STI571 als Inhibitor für Abl. STI571 wird dabei deswegen gegenüber PP1 als Inhibitor für Abl ausgewählt, weil er, anders als PP1, auch die Mutanten T315V und A380T nicht mehr inhibieren kann. Basierend auf den graduellen Veränderungen, welche durch die jeweiligen Mutationen an Position 315 und 380 in Abl vorgenommen wurden (vgl. Fig 8), füllt STI571 den Eingang zur hydrophoben Bindungstasche besser aus.

Durch oben beschriebenes Verfahren wird das gezielte Screening nach Leitstrukturen, die als spezifische Inhibitoren für Enzyme, wie Src-Kinasen oder Abl- Kinase, in Frage kommen, vereinfacht. Durch die Verwendung von Mutanten, bei denen wesentliche Spezifitätsdeterminanten für die Wechselwirkung eines Inhibitors mit der Inhibitor-spezifischen Bindungsstelle eliminiert oder in graduellen Abstufungen verändert wurden, wird der Screening-Prozeß erheblich vereinfacht, da nunmehr gegen die Inhibitor-resistente Mutante gescreent wird und nicht gegenüber einer Vielzahl potentiell kreuzinhibierbarer anderer Kinasen. Die Entwicklungszeiten und -kosten für einen solchen Versuchsaufbau sind insgesamt wesentlich geringer. Darüberhinaus kann bereits auf der Stufe der Lead-Identifikation eine Selektion auf Strukturmerkmale, die potentiellen Inhibitoren eine möglichst hohe Spezifität gegenüber anderen potentiellen Zielmolekülen verleihen, erfolgen.

Neben dem Highthroughput-Screening gewinnt das *structure based, molecular drug design* immer mehr an Bedeutung. Es ist deshalb vorstellbar, basierend auf der Kristallstruktur von Src-Kinasen einerseits und bereits bekannten Leitstrukturen für Tyrosinkinase-Inhibitoren andererseits, modifizierte und optimierte Inhibitoren am Computer zu entwerfen. In Bezug auf Src-Kinasen wäre es dabei sinnvoll, Substanzen zu entwerfen, die die hydrophobe PP1-Bindungstasche möglichst optimal zur Bindung ausnutzen. Mit den so hergestellten Mutanten kann anschließend überprüft werden, inwieweit Vorhersage und Realität übereinstimmen, d.h. ob ein am Computer entworfener und optimierter Inhibitor tatsächlich die PP1-Bindungstasche zur Interaktion mit dem Target nutzt oder nicht.

Wie den beiden vorstehenden Beispielen für die Src-Kinase Hck und die Kinase Abl zu entnehmen, werden die bekannten Inhibitoren für diese beiden Kinasen erfolgreich mit dem erfindungsgemäßen Verfahren erkannt. Insbesondere ein Vergleich des erfindungsgemäßen Verfahrens beim Screenen von PP1 und STI571 gegen Mutanten und das wt-Enzym Abl zeigt, dass mit dem erfindungsgemäßen Verfahren der spezifischere der beiden Inhibitor, STI571, selektiert wird.

Mit Hilfe des erfindungsgemäßen Verfahrens werden Inhibitoren gefunden, die eine hohe Spezifität gegenüber der Inhibierung bestimmter Enzyme haben. Diese können in vielfältigen Bereichen Anwendung finden. Insbesondere können diese als therapeutisches und/oder prophylaktisches Mittel bei der Behandlung von Krankheiten, wie Krebserkrankungen, Allergien, von Abstoßungsreaktionen bei Transplantationen und/oder Osteoporose verwendet werden. Bevorzugt werden mit dem erfindungsgemäßen Mittel Krebserkrankungen, wie Leukämien oder solide Tumoren behandelt.

In einer weiteren Ausführungsform der Erfindung wird ein Verfahren zum Prüfen eines mit dem erfindungsgemäßen Verfahren selektierten Inhibitors auf biologische Wirkungen, die für die Wechselwirkung zwischen dem Inhibitor und der Inhibitor-spezifischen Bindungsstelle spezifisch sind, zur Verfügung gestellt, die Schritte umfassend
1) Inkubieren eines Modellsystems, in dem die wt Proteinkinase exprimiert wird, mit einem Inhibitor,
2) Feststellen der damit erzielten Wirkungen,
3) Inkubieren eines Modellsystems, in dem eine nach Schritt c) des erfindungsgemäßen Verfahrens selektierte Mutante exprimiert wird, mit dem Inhibitor,
4) Feststellen der damit erzielten Wirkungen,
5) Vergleichen der in Schritt 2) und 4) festgestellten Wirkungen, und
6) Auswählen der Wirkungen, die in Schritt 2) festgestellt wurden, nicht aber in Schritt 4), als spezifisch für die Wechselwirkung zwischen dem Inhibitor und der Inhibitor-spezifischen Bindungsstelle.

Das in dem erfindungsgemäßen Verfahren verwendete Modellsystem wird vorzugsweise ausgewählt aus der Gruppe, bestehend aus Zelllinien, Mikroorganismen, und Tieren. Als Tiere können Mäuse, Ratten, oder Kaninchen verwendet werden. Als Zelllinien werden vorzugsweise die Zelllinien 32D und BaF3 , beides Modellsysteme für Leukämie, verwendet.

Die Wirkungen, die in Schritt 2) festgestellt werden, sind vorzugsweise ausgewählt aus der Gruppe, bestehend aus, therapeutischen Wirkungen, akuter und subakuter Organtoxizität, nicht-therapeutischer Immunsuppression und lethalen Wirkungen.

Die vorstehend genannten, Inhibitor-resistenten Mutanten können somit zur weiteren, biologischen Validierung selektierter und optimierter Inhibitoren oder Inhibitor-Leitstrukturen genutzt werden. Durch das erfindungsgemäße Verfahren können Enzym-spezifische Wirkungen des Inhibitors, und somit erwünschte Wirkungen, wie beispielsweise therapuetische Effekte, von Enzym-unabhängigen Wirkungen des Inhibitors, also nicht erwünschten Wirkungen, unterschieden werden.

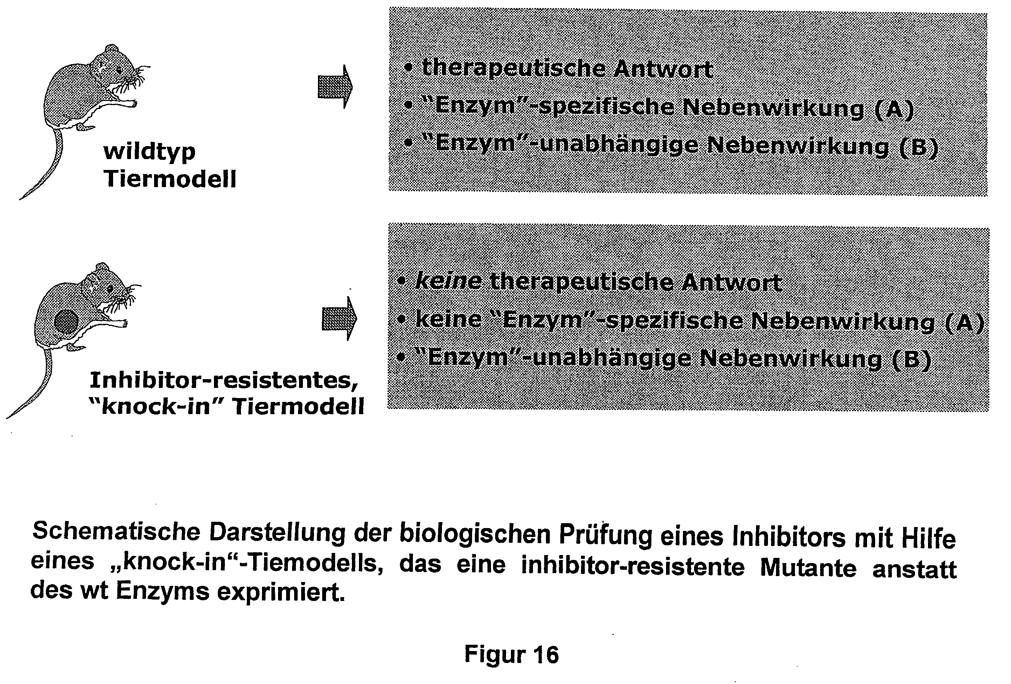

Wie in Figur 16 gezeigt, kann mit diesen Mutanten zwischen Enzym-spezifischen und Enzym-unabhängigen Wirkungen des Inhibitors, insbesondere zwischen Enzym-spezifischen und Enzym-unabhängigen Nebenwirkungen differenziert werden. Im oberen Teil der Figur 16 führt die Inkubation eines Modellsystems, z.B. einer Maus, mit einem Inhibitor zu einem therapeutischen Effekt einerseits, und zu den (Neben-)Wirkungen A und B andererseits. Das Modellsystem kann alternativ auch eine Zellinie sein. Wie im unteren Teil der Figur 16 gezeigt, kann nun in diesem Modellsystem ein Inhibitor-resistentes Allel in einer "knock-in" Maus exprimiert werden. Das entsprechende Genprodukt (IR) ist nicht mehr durch den Inhibitor hemmbar und kann somit auch in dessen Gegenwart seine Funktion erfüllen. Mit diesem Versuchsansatz werden Enzym-spezifischen Wirkungen des Inhibitors aufgehoben. Da Wirkung B nach wie vor auftritt, muss es sich um ein Enzym-unabhängige Wirkung handeln.

Zur Herstellung einer erfindungsgemäß verwendbaren "knock-in" Mäusen werden embryonale Stammzellen (ES-Zellen) kultiviert. In diese Zellen wird ein Transfervektor eingebracht, der neben dem interessierenden Gen (gene of interest) zusätzlich dessen im Genom flankierende Regionen und einen Selektionsmarker, d.h. ein Resistenzgen, enthält. Durch homologe Rekombination kommt es zum Austausch des wt Gens gegen das "knock-in" Gen. Mit Hilfe des Resistenzmarkers werden sodann diejenigen ES-Zellen selektiert, in denen der erwünschte Genaustausch stattgefunden hat. Die so selektierten ES-Zellen werden in Mausblastozysten injeziert und diese in scheinschwangere Mausweibchen implantiert. So werden chimäre Nachkommen erhalten, die schließlich durch Kreuzung zur Erzeugung genetisch reiner Nachkommen verwendet werden.

Durch Verwendung diese Versuchsansatzes (knock-in Strategie) noch vor Beginn klinischer Studien kann eine Differenzierung in Inhibitor-spezifische und Inhibitorunabhängige Wirkungen erfolgen. Dies ist von erheblichem Wert bei der Vorhersage eventueller Nebenwirkungen bei der Verwendung des Inhibitors. Wichtig ist dabei die Entscheidungshilfe, welche das erfindungsgemäße Verfahren im Rahmen der Entwicklung eines Inhibitors, bereitsstellt. Tritt beispielsweise bei der Testung eines Inhibitors in der wildtyp bzw. in der inhibitor-resistenten Maus eine Vielzahl von enzym-unabhängigen, unerwünschten Nebenwirkungen auf, so spricht dies für eine zusätzliche Interaktion des Inhibitors mit einer weiteren Zielstruktur innerhalb des Organismus, die verschieden ist von der therapeutischen Zielstruktur. Demnach muss entweder die Spezifität des Inhibitors durch entsprechende Modifikationen erhöht werden, oder aber es muss eine andere Substanz gefunden werden, welche nicht zu unerwünschten, enzym-unabhängigen Nebenwirkungen führt. Tritt umgekehrt eine Vielzahl enzym-abhängiger Nebenwirkungen auf, so wird das Enzym als therapeutisch-angreifbares Zielstruktur in Frage gestellt. Demnach muss für die entsprechende Krankheit nach einem besseren, therapeutisch-nutzbarem Enzym gesucht werden. Durch diesen Entscheidungs- und Entwicklungsprozess können hohe Kosten für spätere, erfolglose klinische Studien eingespart werden.

Zudem wird mit dem erfindungsgemässen Verfahren die Enzym- bzw. Zielstruktur-Spezifität der therapeutischen Antwort geklärt. Das Wissen um die Enzym- bzw. Zielstruktur-Spezifität der therapeutischen Antwort wiederum kann das Zulassungsverfahren für ein den Inhibitor enthaltendes Medikament wesentlich beschleunigen.

Neben Tiermodellen können auch Zellkulturmodelle zur biologischen Validierung von Inhibitoren mit Hilfe des erfindungsgemäßen Verfahrens verwendet werden.

In einem Ausführungsbeispiel des erfindungsgemäßen Verfahrens wurde entweder Bcr-Abl oder eine zuvor für die Kinase-Inhibitoren PP1 und STI571 als resistent identifizierte Mutante, z.B. T315M in einer murinen, Interleukin-3-abhängigen Zellinie, 32D exprimiert. Bcr-Abl ist eine konstitutiv aktive Tyrosinkinase, die im Tiermodel und bei Menschen verschiedenen Formen von Leukämie induziert.

Zur Durchführung des Ausführungsbeispiels wurden 32D-Zellen mittels Elektroporation, also unter Zuhilfenahme eines Stromimpulses, mit einem Plasmid transfiziert. Dieses Plasmid kodierte entweder für wt Bcr-Abl oder aber z.B. für die Mutante T315M. Zusätzlich tragen diese Plasmide einen Resistenzmarker, in diesem Fall ein Puromycin-Resistenzgen. Transfizierte Zellen wurden daher mit Hilfe von Puromycin selektiert. Nach Selektion wurde ein Teil der Zellen lysiert und mittels Westernblot-Analyse die Expression von Bcr-Abl nachgewiesen.

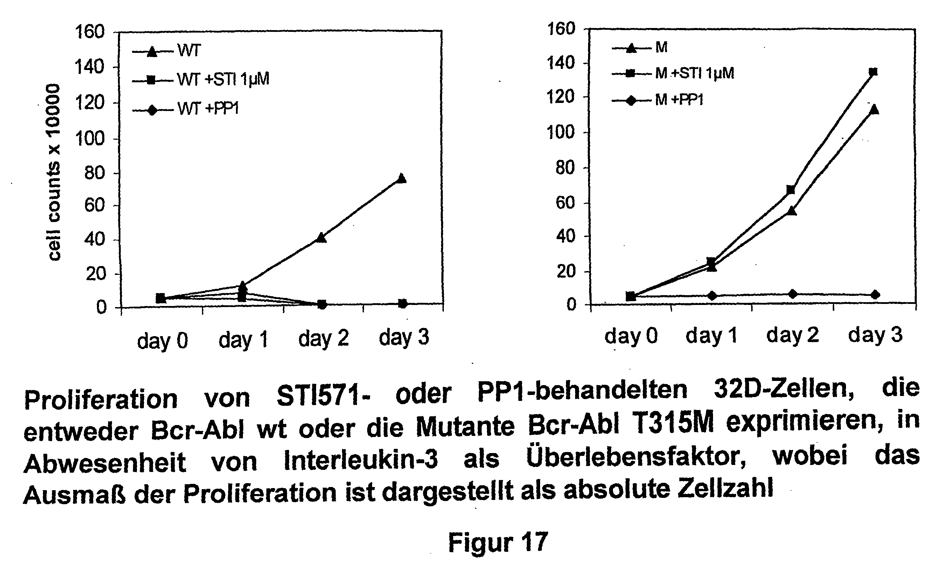

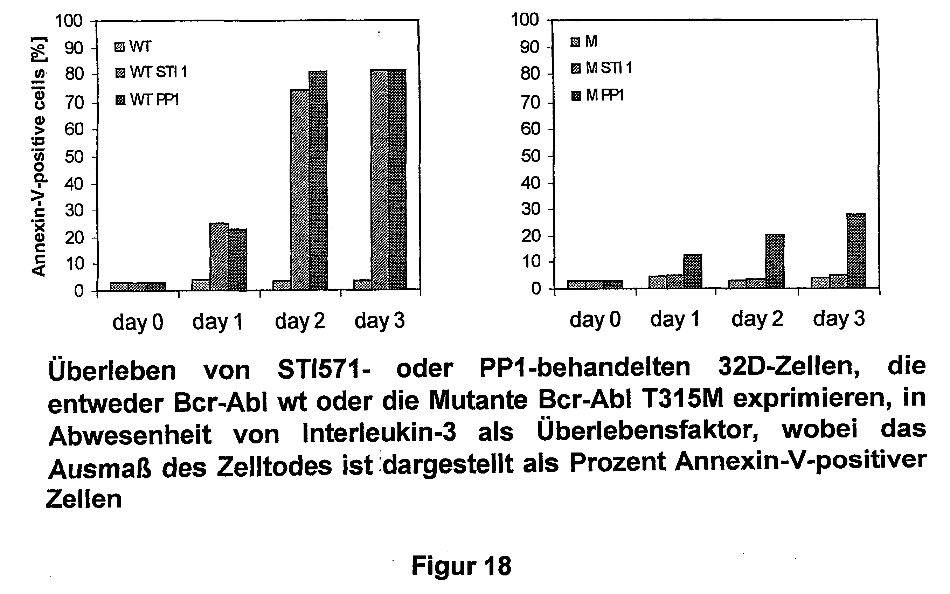

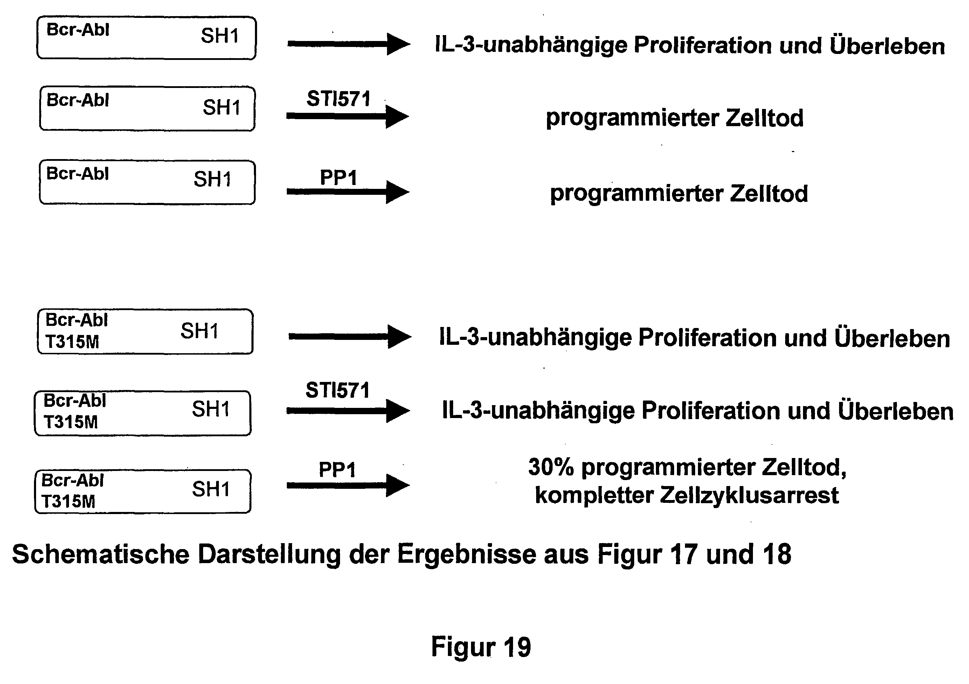

Anschliessend wurden die Zellen biologisch charakterisiert. Dazu wurde den Zellen Interleukin-3 als essentieller Überlebens- und Wachstumsfaktor entzogen. Während untransfizierte 32D-Zellen unter Interleukin-3-Entzug aufhören zu proliferieren und innerhalb von 24h absterben, konnten sowohl Zellen die mit wt Bcr-Abl, als auch Zellen, die mit einer Mutante von Bcr-Abl an Position 315, beispielsweise T315M, transfiziert worden waren, auch ohne Gegenwart von Interleukin-3 im Kulturmedium überleben und proliferieren (Fig. 17 und 18) , und zwar in gleichem Masse. Dies zeigt, daß die Mutation von Bcr-Abl an der Position 315, beispielsweise zu Methionin, die leukämogene Potenz der Kinase nicht aufhebt oder reduziert (Fig.17).

Zusätzlich wurde zu einem Teil der Zellen entweder PP1 (25µM) oder STI571 (1µM) gegeben. Beide Substanzen verhinderten sowohl die Proliferation als auch das Überleben von Zellen, die Bcr-Abl wt exprimierten, und zwar in etwa gleichem Ausmaß (Fig.17 und 18).

Diejenigen Zellen, welche eine Mutante von Bcr-Abl an Position 315, beispielsweise T315M, exprimierten und mit STI571 inkubiert wurden, zeigten Proliferation und Überleben wie unbehandelte Zellen (Fig.17 und 18). In diesen Zellen kann STI571 Bcr-Abl nicht inhibieren, weil durch die Verlängerung der Aminosäureseitenkette an Position 315 der Zugang zur Inhibitorspezifischen Bindungsstelle blockiert wird. Das Ausbleiben eines biologischen Effektes von STI571 in diesen Zellen belegt daher, dass Bcr-Abl das einzige wirkungsrelevante Zielmolekül von STI571 unter diesen Bedingungen ist. Die Wirkung von STI571 ist also "Zielmolekül"-spezifisch. Dies lässt auf eine geringe Toxizität der Substanz schliessen.

Im Gegensatz dazu führte die Inkubation von Zellen, welche die Mutante T315M exprimierten, mit PP1 zu einem kompletten Stop des Zellwachstums und zu einer reduzierten Überlebensrate der Zellen (Fig.17 und 18). Insgesamt kam es zu einem Absterben von ca. 30% der Zellen. Da die dabei verwendete Mutante T315M biochemisch resistent gegenüber PP1 ist (s.o.), zeigt dieses Ergebnis, dass PP1 in Bcr-Abl-positiven 32D Zellen neben Bcr-Abl noch eine weitere biologisch relevante Zielstruktur erkennt, die zu einer unspezifischen Toxizität führt. Es ist also davon auszugehen, dass PP1 in vivo eine vergleichsweise höhere Toxizität besitzt als STI571.

Im Schritt 6 des erfindungsgemäßen Verfahrens wird hier demnach STI571 als Ablspezifischer Inhibitor selektiert, weil seine biologische Wirkung auf Bcr-Ablexprimierende 32D-Zellen rein "zielmolekül"-spezifisch ist. PP1 hemmt zwar ebenfalls Bcr-Abl und führt zum Absterben der Zellen, jedoch ist diese Wirkung nicht absolut "Zielmolekül"-spezifisch, so dass in vivo mit einer größeren Toxizität dieses Inhibitors zu rechnen ist. In der Tat wird derzeit STI571 in klinischen Studien getestet, ohne dass dabei bisher Zeichen einer relevanten Toxizität aufgetreten sind.

Um zu Überprüfen, ob es sich bei dem von Bcr-Abl verschiedenen, biologisch relevanten Zielmolekül für PP1 in 32D Zellen um eine Tyrosinkinase handelt, wurden Zellen die entweder wt Bcr-Abl exprimierten oder die Mutante T315M unbehandelt gelassen oder aber mit STI571 (1µM) bzw. PP1 (25µM) behandelt und anschliessend lysiert. Die so gewonnenen Proteinextraktewurden anschließend auf Tyrosinphosphorylierung zellulärer Proteine untersucht. Dies geschah unter Verwendung des Antikörpers PY99, der spezifisch tyrosinphosphorylierte Proteine erkennt. Dabei zeigte sich, dass sowohl Bcr-Abl wt als auch die Mutante T315M die Phosphorylierung zahlreicher zellulärer Proteine induzierten. Die Inkubation von Zellen, welche Bcr-Abl wt exprimierten mit entweder STI571 oder PP1 führte zu einer in etwa gleichmässigen Reduktion der Tyrosinphosphorylierung von Bcr-Abl und anderer zellulärer Proteine. Die Inkubation der Mutante T315M mit STI571 führte zu keiner Auswirkung auf die Phosphorylierung zellulärer Proteine, d.h. es bestand eine komplette Resistenz gegenüber STI571. Wurden jedoch Zellen, welche die Mutante T315M exprimierten, mit PP1 inkubiert, so kam es zu einer deutlichen Abnahme der Phosphorylierung einiger zellulärer Proteine. Dies deutet darauf hin, dass PP1 in diesen Zellen auf eine weitere, biologisch relevante Tyrosinkinase wirkt. Hierbei könnte es sich beispielsweise um Kinasen aus der Src-Familie handeln, die ebenso durch PP1 gehemmt werden (s.o.). Es ist aus der Literatur bekannt, dass STI571 Src-Kinasen nicht inhibiert.

Das erfindungsgemässe Verfahren kann demnach auch zur detaillierten molekularen Analyse Enzym-unspezifischer Wirkungen eingesetzt werden.

Nachstehend wird ein Verfahren zum Durchführen des erfindungsgemäßen Verfahrens genauer beschrieben.

### Zellkultur

Cos7 Zellen wurden in Dulbecco's modified Eagle's Medium (DMEM), das mit 10% fetalem Kälberserum (FKS) angereichert wurde, kultiviert.

### Plasmide und Herstellung von Punktmutanten

Zur Herstellung von Punktmutanten wurde die cDNA des humanen Hck-Gens in den Vektor pUC18 kloniert. Dieses Plasmid diente anschließend als Vorlage für eine mutagene PCR-Reaktion. Die PCR wurde unter Verwendung mutagener Primer durchgeführt, die an ihren 5'-Enden phosphoryliert waren und so kostruiert waren, dass sie an unmittelbar benachbarten Regionen banden. Mit Hilfe von Standard-Einstellungen (Annealing-Temperatur entsprechend der Primer) wurde so das komplette Plasmid amplifiziert, wobei als Polymerase Pfu (Promega) verwendet wurde. Anschließend wurde das lineare PCR-Amplifikat aufgereinigt, mit Hilfe von T4-DNA-Ligase zu einem Plasmid religiert und klonal in Bakterien vemehrt. Die so hergestellten Mutanten wurden schließlich auf Richtigkeit der Mutation hin untersucht.

Zur Expression in Cos7-Zellen wurden die mutierten Hck-Allele in die EcoRI-Schnittstelle des Vektors pApuro kloniert. Dieser Vektor leitet sich von pBabepuro ab, besitzt aber einen Hühnchen-Aktin-Promotor.

### Transfektion von Cos7-Zellen mit Effecten

Für die Transfektion der Hck-Mutanten in Cos7-Zellen wurden diese 18 - 24 h vor Beginn der Transfektion frisch ausgesäht, so dass mit Beginn der Transfektion eine Zelldichte von 50 - 75% erreicht war. Die Transfektion selbst wurde mit Hilfe des Transfektionsreagenzes Effecten (Qiagen) durchgeführt. Dazu wurden 1µg DNA in 150µl Puffer PB aufgenommen und kurz vermischt. Anschließend wurden 8µl des Enhancers zupipettiert. Der Ansatz wurde erneut vermischt und für 2 min bei Raumtemperatur inkubiert. Danach wurden 10µl des Transfektionreagenzes Effecten dazugegeben, der Ansatz vermischt und für 10 min bei Raumtemperatur inkubiert. Der komplette Ansatz wurde in DMEM/10%FKS aufgenommen und vorsichtig auf die zu transfizierenden Zellen pipettiert. Nach kurzem und vorsichtigem Schütteln wurden die Zellen in einen CO₂-Inkubator verbracht und für insgesamt 48 h inkubiert, wobei 24 Stunden nach Beginn der Transfektion ein Medienwechsel durchgeführt wurde. 48 h nach Beginn der Transfektion wurden die Zellen lysiert

### Behandlung von Cos7-Zellen mit PP1

Zur Untersuchung des Effektes von PP1 auf die Aktivität von wildtyp Hck und den verschiedenen Mutanten in vivo wurde 4h vor Beginn der Zellyse das Medium entsprechend transfizierter Zellen abgenommen und durch DMEM/10%FKS, das 25µM PP1 in Dimethylsulfoxid (DMSO) enthielt, ersetzt. Zur Kontrolle wurden Zellen mit einer äquivalenten Menge DMSO ohne PP1 inkubiert.
PP1 wurde von Alexis erworben und als 25mM Stocklösung bei 4°C für maximal 4 Wochen gelagert.

### Lyse von Cos7-Zellen

Zur Lyse wurde das Medium abgenommen und die Cos7-Zellen wurden mit 3 ml Trypsin-EDTA-Lösung vom Boden der Kulturflasche gelöst. Danach wurden die Zellen mit DMEM/10% FKS in ein 50 ml-Zentrifugenröhrchen überführt und abzentrifugiert.

Zur Lyse der Cos7-Zellen wurde das Zellpellet in 250 µl Lysispuffer aufgenommen (1% NP-40, 20 mM Tris (pH8,0), 50 mM NaCl, and 10 mM EDTA, 1 mM PMSF, 10 µg/ml Aprotinin, 10 µg/ml Leupeptin und 2 mM Natriumorthovanadat.) und in ein 1,5ml Eppendorf-Reagiergefäß überführt. Der Ansatz wurde kurz mit einem Vortex gemischt und 30 min bei 4°C auf einem Überkopf-Rotor inkubiert. Danach wurde das Zellysat 15 Min. bei 4°C und 14000 Umdrehungen pro min (rpm) abzentrifugiert. Zuletzt wurde der Überstand, der die zytoplasmatischen Proteine enthielt, in ein neues Reaktionsgefäß überführt.

### SDS-Page und Western-Blot

Zur Auftrennung der Proteine der oben gewonnenen Zellysate wurden mittels SDS-Polyacrylamid-Elektrophorese wurden 80µg des Lysats 1:1 mit 2x Proben-Puffer versetzt und 5 Min. bei 100°C denaturiert. Die Proben wurden komplett in die Taschen des Gels geladen. Nach Auftrennung der Proteine im elektrischen Feld wurden diese auf eine Nitrocellulose-Membran transferriert. Diese wurde anschließend für mindestens 1h in einer Plastikschale mit 15ml TBS, das 5% Magermilchpulver enthielt, inkubiert. Anschließend wurde die Membran 2 - 3 mal mit TBS gespült. Danach wurde die Membran 2 - 18 h mit dem primären Antikörper (anti-Phosphotyrosin PY99 der Firma Santa Cruz Biotch., anti-Hck N-30 der Firma Santa Cruz Biotech. in Verdünnungen von jeweils 1:1000) inkubiert. Die Verdünnung der Antikörper erfolgte in 15ml TBS/1%Magermilchpulver. Anschließend wurde die Membran dreimal 5 min. mit TBS gewaschen. Schließlich wurde die Membran für 1h mit sekundärem Antikörper (Esel-anti-Maus oder Esel-anti-Kanninchen-Antikörper an Merrettich-Peroxidas gekoppelt in einer Verdünnung von 1:2000; beide Antikörper von Amersham) inkubiert und anschließend erneut dreimal 5 min mit TBS gespült.

Zur Detektion der mit Antikörper markierten Proteine wurden jeweils 1 ml der ECLTM-Detektionsreagenzien 1 und 2 (Amersham) gemischt und in der Dunkelkammer auf die in einer Plastikschale befindliche Membran gegossen. Nach genau 1 min. wurde die Detektionslösung abgegossen, die Membran herausgenommen, gut abgetropft und mit Saran-Verpackungsfolie blasenfrei abgedeckt. Schließlich wurden ECLTM- Hyperfilme für 3 - 60 Sek. aufgelegt und in einem geeigneten Entwicklungsapparat (Agfa) entwickelt.

## Patentansprüche

1. Verfahren zum Selektieren von Inhibitoren, die Schritte umfassend
a) Bestimmen einer Inhibitor- nicht aber Substrat-spezifischen Bindungsstelle in einer wt Proteinkinase,
b) Austausch mindestens einer Aminosäure an der als Inhibitor-spezifisch bestimmten Bindungsstelle der wt Proteinkinase durch eine davon verschiedene Aminosäure, wodurch eine Mutante der wt Proteinkinase erhalten wird,
c) Prüfen der in Schritt b) erhaltenen Mutanten auf Enzym-Aktivität und Auswählen der aktiven Mutanten,
d) Prüfen mindestens einer Substanz mit der wt Proteinkinase und der in Schritt c) ausgewählten Mutante, und
e) Auswählen der Substanz als Inhibitor, wenn sie die wt Proteinkinase inhibiert, nicht aber die in Schritt c) ausgewählte Mutante.

2. Verfahren nach Anspruch 1, wobei zusätzlich in Schritt c) die Mutante auf Hemmbarkeit durch einen bekannten Inhibitor geprüft wird und die Mutante ausgewählt wird, die Inhibitor-resistent ist.

3. Verfahren nach einem der vorstehenden Ansprüche, wobei die Protein-Kinase ausgewählt ist aus der Gruppe, bestehend aus den Src-Kinasen Src, Lyn, Fyn, Hck, Lck, Blk, Yes, Yrk, Fgr, den Kinasen ZAP70, BTK, Tec, Jak1, Jak2, PKA, VEGF-Familie-, PDGF-Familie und EGF-Familie-Rezeptorkinasen, MAP-Kinasen, c-Abl und Zyklin-abhängigen Kinasen.

4. Verfahren nach einem der vorstehenden Ansprüche, wobei die Protein-Kinase eine Src-Kinase ist.

5. Verfahren nach Anspruch 4, wobei die Src-Kinase die Src-Kinase Hck ist.

6. Verfahren nach einem der Ansprüche 1 bis 3, wobei die Proteinkinase die Tyrosinkinase Abl ist.

7. Verfahren nach einem der vorstehenden Ansprüche, wobei in Schritt b) die Aminosäure durch eine sterisch aufwendigere, hydrophobere, hydrophilere, basischere oder azidere Aminosäure ausgetauscht wird.

8. Verfahren nach einem der Ansprüche 4 oder 5, wobei in Schritt b) Threonin an der Position 338, Alanin an der Position 403, Leucin an der Position 325, Methionin an der Position 314, und/oder Isoleucin an der Position 336 durch eine davon verschiedene Aminosäure ausgetauscht wird.

9. Verfahren nach Anspruch 8, wobei Threonin an der Position 338 und Alanin an der Position 403 durch eine davon verschiedene Aminosäure ausgetauscht werden.

10. Verfahren nach Anspruch 9, wobei Threonin an der Position 338 ausgetauscht wird durch eine Aminosäure, ausgewählt aus der Gruppe, bestehend aus Valin, Leucin, Isoleucin, Methionin, Glutamin und Phenylalanin, und/oder Alanin an der Position 403 ausgetauscht wird durch eine Aminosäure, ausgewählt aus der Gruppe, bestehend aus Serin, Cystein und Threonin.

11. Verfahren nach Anspruch 8, wobei Methionin an der Position 314, Leucin an der Position 325 und/oder Isoleucin an der Position 336 durch Phenylalanin und/oder Threonin ausgetauscht wird.

12. Verfahren nach Anspruch 6, wobei Threonin an der Position 315 ausgetauscht wird durch eine Aminosäure, ausgewählt aus der Gruppe, bestehend aus Valin, Leucin, Isoleucin, Methionin, Glutamin und Phenylalanin und/oder Alanin an der Position 380 ausgetauscht wird durch eine Aminosäure, ausgewählt aus der Gruppe, bestehend aus Serin, Cystein und Threonin.

13. Verfahren nach Anspruch 6, wobei Methionin an der Position 290, Leucin an der Position 301 und/oder Isoleucin an der Position 313 durch Phenylalanin und/oder Threonin ausgetauscht wird.

14. Verfahren nach einem der vorstehenden Ansprüche, wobei in Schritt e) eine Vielzahl von Substanzen gleichzeitig geprüft wird.

15. Verfahren zum Prüfen eines mit einem Verfahren nach einem der Ansprüche 1 bis 14 selektierten Inhibitors auf biologische Wirkungen, die für die Wechselwirkung zwischen dem Inhibitor und der Inhibitor-spezifischen Bindungsstelle spezifisch sind, die Schritte umfassend
1) Inkubieren eines Modellsystems, in dem die wt Proteinkinase exprimiert wird, mit einem Inhibitor,
2) Feststellen der damit erzielten Wirkungen,
3) Inkubieren eines Modellsystems, in dem eine nach Schritt c) des Verfahrens nach einem der Ansprüche 1 bis 14 selektierten Mutante exprimiert wird, mit dem Inhibitor,
4) Feststellen der damit erzielten Wirkungen,
5) Vergleichen der in Schritt 2) und 4) festgestellten Wirkungen, und
6) Auswählen der Wirkungen, die in Schritt 2) festgestellt wurden, nicht aber in Schritt 4), als spezifisch für die Wechselwirkung zwischen dem Inhibitor und der Inhibitor-spezifischen Bindungsstelle.

16. Verfahren nach Anspruch 15, wobei das Modellsystem eine Zelllinie, Mikroorganismus oder Tier ist.

17. Verfahren nach Anspruch 16, wobei das Tier ausgewählt ist aus der Gruppe, bestehend aus Mäusen, Ratten und Kaninchen.

18. Verfahren nach einem der Ansprüche 15 bis 17, wobei die Wirkungen ausgewählt sind aus der Gruppe, bestehend aus, therapeutischen Wirkungen, Organtoxizität, nicht-therapeutischer Immunsuppression und lethalen Wirkungen.

## Claims

1. A method of selecting inhibitors, comprising the steps of
a) determining a binding site in a wt protein kinase which is inhibitor-specific but not substrate-specific;
b) replacing at least one amino acid at the binding site of the wt protein kinase which is found to be inhibitor-specific with a different amino acid, whereby a mutant of the wt protein kinase is obtained;
c) testing the mutants obtained in step b) for enzyme activity and selecting the active mutants;
d) testing at least one substance with the wt protein kinase and the mutant selected in step c); and
e) selecting the substance as inhibitor if it inhibits the wt protein kinase but not the mutant selected in step c).

2. The method according to claim 1, wherein additionally in step c) the mutant is tested as to whether it can be inhibited by a known inhibitor, and the mutant which is inhibitor-resistant is selected.

3. The method according to any one of the preceding claims, wherein the protein kinase is selected from the group consisting of the Src kinases Src, Lyn, Fyn, Hck, Lck, Blk, Yes, Yrk, Fgr, the kinases ZAP70, BTK, Tec, Jak1, Jak2, PKA, VEGF-family, PDGF-family and EGF-family receptor kinases, MAP kinases, c-Abl and cyclin-dependent kinases.

4. The method according to any one of the preceding claims, wherein the protein kinase is an Src kinase.

5. The method according to claim 4, wherein the Src kinase is the Src kinase Hck.

6. The method according to any one of claims 1 to 3, wherein the protein kinase is tyrosine kinase Abl.

7. The method according to any one of the preceding claims, wherein in step b) the amino acid is substituted by an amino acid which takes up more space or is more hydrophobic, more hydrophilic, more basic or more acid.

8. The method according to any one of claims 4 or 5, wherein in step b) threonine at position 338, alanine at position 403, leucine at position 325, methionine at position 314, and/or isoleucine at position 336 is substituted by a different amino acid.

9. The method according to claim 8, wherein threonine at position 338 and alanine at position 403 are substituted by a different amino acid.

10. The method according to claim 9, wherein threonine at position 338 is substituted by an amino acid selected from the group consisting of valine, leucine, isoleucine, methionine, glutamine and phenylalanine, and/or alanine at position 403 is substituted by an amino acid selected from the group consisting of serine, cysteine and threonine.

11. The method according to claim 8, wherein methionine at position 314, leucine at position 325 and/or isoleucine at position 336 is substituted by phenylalanine and/or threonine.

12. The method according to claim 6, wherein threonine at position 315 is substituted by an amino acid selected from the group consisting of valine, leucine, isoleucine, methionine, glutamine and phenylalanine and/or alanine at position 380 is substituted by an amino acid selected from the group consisting of serine, cysteine and threonine.

13. The method according to claim 6, wherein methionine at position 290, leucine at position 301 and/or isoleucine at position 313 is substituted by phenylalanine and/or threonine.

14. The method according to any one of the preceding claims, wherein a plurality of substances are tested simultaneously in step e).

15. A method of testing an inhibitor selected by a method according to any one of claims 1 to 14, for biological effects specific to the interaction between the inhibitor and the inhibitor-specific binding site, comprising the steps of
1) incubating a modeling system in which the wt protein kinase is expressed, with an inhibitor,
2) establishing the effects thereby obtained,
3) incubating a modeling system, in which a mutant selected according to step c) of the method according to any one of claims 1 to 14 is expressed, with the inhibitor,
4) establishing the effects thereby obtained,
5) comparing the effects found in step 2) and 4), and
6) selecting the effects found in step 2) but not in step 4), as being specific to the interaction between the inhibitor and the inhibitor-specific binding site.

16. The method according to claim 15, wherein the modeling system is a cell line, microorganism or animal.

17. The method according to claim 16, wherein the animal is selected from the group comprising mice, rats and rabbits.

18. The method according to any one of claims 15 to 17, wherein the effects are selected from the group consisting of therapeutic effects, organ toxicity, non-therapeutic immunosuppression and lethal effects.

## Revendications

1. Procédé pour sélectionner des inhibiteurs, qui comprend les étapes suivantes :
a) détermination d'un site de liaison, spécifique d'inhibiteur mais non spécifique de substrat, dans une protéine kinase de type sauvage,
b) remplacement d'au moins un acide aminé au niveau du site de liaison, défini comme étant spécifique d'inhibiteur, de la protéine kinase de type sauvage, par un acide aminé qui en est différent, ce qui permet d'obtenir un mutant de la protéine kinase de type sauvage,
c) vérification de l'activité enzymatique des mutants obtenus dans l'étape b), et sélection des mutants actifs,
d) contrôle d'au moins une substance avec la protéine kinase de type sauvage et le mutant sélectionné dans l'étape c), et
e) sélection de la substance en tant qu'inhibiteur si elle inhibe la protéine kinase de type sauvage, mais non pas le mutant sélectionné dans l'étape c).

2. Procédé selon la revendication 1, **caractérisé en ce que** dans l'étape c), le mutant fait en outre l'objet d'un contrôle de son aptitude à être inhibé par un inhibiteur connu, et le mutant qui résiste à l'inhibiteur est sélectionné.

3. Procédé selon l'une des revendications précédentes, dans lequel la protéine kinase est choisie dans l'ensemble consistant en les Src-kinases Src, Lyn, Fyn, Hck, Lck, Blk, Yes, Yrk, Fgr, les kinases ZAP70, BTK, Tec, Jak1, Jak2, PKA, les récepteurs-kinases de la famille VEGF, de la famille PDGF et de la famille EGF, les MAP-kinases, le c-Abl, et les kinases qui dépendent de la cycline.

4. Procédé selon l'une des revendications précédentes, dans lequel la protéine kinase est une Src-kinase.

5. Procédé selon la revendication 4, dans lequel la Src-kinase est la Src-kinase Hck.

6. Procédé selon l'une des revendications 1 à 3, dans lequel la protéine kinase est la tyrosine kinase Abl.

7. Procédé selon l'une des revendications précédentes, dans lequel, dans l'étape b), l'acide aminé est remplacé par un acide aminé stériquement plus encombré, plus hydrophobe, plus hydrophile, plus basique ou plus acide.

8. Procédé selon l'une des revendications 4 et 5, dans lequel, dans l'étape b), la thréonine en position 338, l'alanine en position 403, la leucine en position 325, la méthionine en position 314 et/ou l'isoleucine en position 336 est/sont remplacée(s) par un acide aminé qui en est différent.

9. Procédé selon la revendication 8, dans lequel la thréonine en position 338 et l'alanine en position 403 sont remplacées par un acide aminé qui en est différent.

10. Procédé selon la revendication 9, dans lequel la thréonine en position 338 est remplacée par un acide aminé choisi dans le groupe consistant en la valine, la leucine, l'isoleucine, la méthionine, la glutamine et la phénylalanine, et/ou l'alanine en position 403 est remplacée par un acide aminé choisi dans le groupe consistant en la sérine, la cystéine et la thréonine.

11. Procédé selon la revendication 8, dans lequel la méthionine en position 314, la leucine en position 325 et/ou l'isoleucine en position 336 est/sont remplacée(s) par la phénylalanine et/ou la thréonine.

12. Procédé selon la revendication 6, dans lequel la thréonine en position 315 est remplacée par un acide aminé choisi dans le groupe consistant en la valine, la leucine, l'isoleucine, la méthionine, la glutamine et la phénylalanine, et/ou l'alanine en position 380 est remplacée par un acide aminé choisi dans le groupe consistant en la sérine,.: la cystéine et la thréonine.

13. Procédé selon la revendication 6, dans lequel. la méthionine en position 290, la leucine en position 301 et/ou l'isoleucine en position 313 est/sont remplacée(s) par la phénylalanine et/ou la thréonine.

14. Procédé selon l'une des revendications précédentes, dans lequel, dans l'étape e), on contrôle simultanément un grand nombre de substances.

15. Procédé pour contrôler, sur un inhibiteur sélectionné par un procédé selon l'une des revendications 1 à 14, les effets biologiques qui sont spécifiques de l'interaction entre l'inhibiteur et le site de liaison spécifique d'inhibiteur, comprenant les étapes suivantes :
1) incubation d'un système modèle dans lequel la protéine kinase de type sauvage est exprimée, avec un inhibiteur,
2) détermination des effets ainsi obtenus,
3) incubation, avec l'inhibiteur, d'un système modèle dans lequel est exprimé un mutant sélectionné selon l'étape c) du procédé selon l'une des revendications 1 à 14,
4) détermination des effets ainsi obtenus,
5) comparaison des effets constatés dans l'étape 2) et l'étape 4), et
6) sélection des effets qui ont été constatés dans l'étape 2) mais non dans l'étape 4), comme étant spécifiques de l'interaction entre l'inhibiteur et le site de liaison spécifique d'inhibiteur.

16. Procédé selon la revendication 15, dans lequel le système modèle est une lignée cellulaire, un microorganisme ou un animal.

17. Procédé selon la revendication 16, dans lequel l'animal est choisi dans le groupe consistant en les souris, les rats et les lapins.

18. Procédé selon l'une des revendications 15 à 17, dans lequel les effets sont choisis dans le groupe consistant en des effets thérapeutiques, la toxicité d'organe, l'immunosuppression non thérapeutique et des effets létaux.
